# EUROPEAN PATENT SPECIFICATION

(11) **EP 2 938 353 B1**
(45) Date of publication and mention of the grant of the patent: **15.08.2018**
(21) Application number: 13831825.8
(22) Date of filing: 26.12.2013
(51) Int. Cl.: A61K 38/16

(54) **ANTI-INFLAMMATORY PEPTIDES**
ENTZÜNDUNGSHEMMENDE PEPTIDE
PEPTIDES ANTI-INFLAMMATOIRES

(30) Priority: 26.12.2012 EP 12008611
(43) Date of publication of application: 04.11.2015
(73) Proprietor: Institut National de la Recherche Agronomique (INRA), 75338 Paris Cedex 07 (FR)
(72) Inventor: LANGELLA Philippe, 78140 Vélizy-Villacoublay (FR); PIGNEUR-ARNAUD, Bénédicte, 75015 Paris (FR); CHATEL, Jean-Marc, 92190 Meudon (FR); TRUGNAN, Germain, 93100 Montreuil (FR); QUEVRAIN, Elodie, 94170 Le Perreux sur Marne (FR); SEKSIK, Philippe, 75011 Paris (FR); BERMUDEZ-HUMARAN, Luis G., 78350 Jouy en Josas (FR); CHAIN, Florian, 78000 Versailles (FR); MAUBERT, Marie-Anne, 75019 Paris (FR); SOKOL, Harry, 75018 Paris (FR); MICHON, Christophe, 93190 Livry-Gargan (FR)
(74) Representative: Barbot, Willy
(86) International application number: PCT/EP2013/003917
(87) International publication number: WO 2014/102009

(56) References cited:
- SOKOL HARRY ET AL: "Faecalibacterium prausnitzii is an anti-inflammatory commensal bacterium identified by gut microbiota analysis of Crohn disease patients", PROCEEDINGS OF THE NATIONAL ACADEMY OF SCIENCES, NATIONAL ACADEMY OF SCIENCES, US, vol. 105, no. 43, 28 October 2008 (2008-10-28), pages 16731-16736, XP002579466, ISSN: 0027-8424, DOI: 10.1073/PNAS.0804812105 [retrieved on 2008-10-20]
- DATABASE BIOSIS [Online] BIOSCIENCES INFORMATION SERVICE, PHILADELPHIA, PA, US; May 2012 (2012-05), ROSIQUE REBECA MARTIN ET AL: "Protective and Curative Effect of Faecalibacterium prausnitzii in a Chronic DNBS-Induced Murine Colitis", XP002695881, Database accession no. PREV201200606260 & GASTROENTEROLOGY, vol. 142, no. 5, Suppl. 1, May 2012 (2012-05), page S392, DIGESTIVE DISEASE WEEK (DDW); SAN DIEGO, CA, USA; MAY 19 -22, 2012 ISSN: 0016-5085(print)

### SEQUENCE LISTING

<110> INRA
<120> ANTI-INFLAMMATORY PEPTIDES
<130> INR-B-00016-PCT1
<150> EP12008611.1
   <151> 2012-12-26
<160> 22
<170> PatentIn version 3.5
<210> 1
   <211> 166
   <212> PRT
   <213> Artificial Sequence
<220>
   <223> Consensus sequence
<220>
   <221> x
   <222> (7)..(7)
   <223> X= Phe or Tyr
<220>
   <221> X
   <222> (8)..(8)
   <223> x= Ser or Thr
<220>
   <221> X
   <222> (9)..(9)
   <223> X= Ala or Val
<220>
   <221> X
   <222> (10)..(10)
   <223> X= val or Ile
<220>
   <221> X
   <222> (11)..(11)
   <223> X= Ser, Ala or Asn
<220>
   <221> X
   <222> (12)..(12)
   <223> X= Glu or no amino acid
<220>
   <221> X
   <222> (13)..(13)
   <223> X= Asn or no amino acid
<220>
   <221> X
   <222> (14)..(14)
   <223> x= Glu or no amino acid
<220>
   <221> X
   <222> (15)..(15)
   <223> X= Met, Ser or Ala
<220>
   <221> X
   <222> (16)..(16)
   <223> X= Thr or Glu
<220>
   <221> x
   <222> (17)..(17)
   <223> X= Tyr or Val
<220>
   <221> X
   <222> (19)..(19)
   <223> X= Met, Val, Asn or Tyr
<220>
   <221> X
   <222> (22)..(22)
   <223> X= Ser or Ala
<220>
   <221> X
   <222> (23)..(23)
   <223> x= Asn, Asp or no amino acid
<220>
   <221> X
   <222> (24)..(24)
   <223> X= Phe, Leu or no amino acid
<220>
   <221> x
   <222> (25)..(25)
   <223> X= Ile, Phe or no amino acid
<220>
   <221> X
   <222> (26)..(26)
   <223> X= Asp, Thr or no amino acid
<220>
   <221> X
   <222> (27)..(27)
   <223> X= Ala, Ile or no amino acid
<220>
   <221> X
   <222> (28)..(28)
   <223> x= val, Leu or Ile
<220>
   <221> X
   <222> (29)..(29)
   <223> X= Ala, Val or Gly
<220>
   <221> X
   <222> (30)..(30)
   <223> x= Ala or Asp
<220>
   <221> X
   <222> (31)..(31)
   <223> X= Tyr, Val or Thr
<220>
   <221> X
   <222> (32)..(32)
   <223> X= Leu or Thr
<220>
   <221> X
   <222> (33)..(33)
   <223> X= Ala or Pro
<220>
   <221> x
   <222> (34)..(34)
   <223> X= Pro or Ser
<220>
   <221> X
   <222> (35)..(35)
   <223> X= Ala or Ile
<220>
   <221> X
   <222> (36)..(36)
   <223> X= Met or Trp
<220>
   <221> X
   <222> (37)..(37)
   <223> X= Gly, Thr or Asn
<220>
   <221> X
   <222> (38)..(38)
   <223> X= Ala, Thr or Leu
<220>
   <221> X
   <222> (39)..(39)
   <223> X= Ala, Asp or Glu
<220>
   <221> X
   <222> (40)..(40)
   <223> X= Gln, Asn or Ser
<220>
   <221> X
   <222> (41)..(41)
   <223> X= Trp or Val
<220>
   <221> X
   <222> (42)..(42)
   <223> X= Gln or Lys
<220>
   <221> X
   <222> (43)..(43)
   <223> X= Asn, Thr, Lys or Arg
<220>
   <221> X
   <222> (44)..(44)
   <223> X= Phe or Val
<220>
   <221> X
   <222> (45)..(45)
   <223> X= His, Ser, Asn
<220>
   <221> X
   <222> (46)..(46)
   <223> X= Lys, Ala, Thr or ser
<220>
   <221> X
   <222> (48)..(48)
   <223> X= Leu, Val or Ile
<220>
   <221> X
   <222> (49)..(49)
   <223> X= Ile or Val
<220>
   <221> X
   <222> (50)..(50)
   <223> X= Thr or Lys
<220>
   <221> X
   <222> (51)..(51)
   <223> X= Ile or Leu
<220>
   <221> X
   <222> (52)..(52)
   <223> X= Val or Ile
<220>
   <221> X
   <222> (53)..(53)
   <223> X= Gly or Ser
<220>
   <221> X
   <222> (55)..(55)
   <223> X= Lys, Ser or Thr
<220>
   <221> X
   <222> (56)..(56)
   <223> X= Tyr or Phe
<220>
   <221> X
   <222> (57)..(57)
   <223> X= Val, Leu, Phe or Thr
<220>
   <221> X
   <222> (58)..(58)
   <223> X= Gln, Ala, Lys or Ser
<220>
   <221> X
   <222> (59)..(59)
   <223> x= Gly, Lys, Ser or His
<220>
   <221> X
   <222> (60)..(60)
   <223> X= Phe, Tyr, Thr or Leu
<220>
   <221> X
   <222> (61)..(61)
   <223> X= Leu, Thr, Ile, or val
<220>
   <221> X
   <222> (62)..(62)
   <223> X=Asp, Asn, Ser or Lys
<220>
   <221> X
   <222> (63)..(63)
   <223> X= Asn, Asp, Arg or Ala
<220>
   <221> X
   <222> (64)..(64)
   <223> X= Thr or Val
<220>
   <221> X
   <222> (65)..(65)
   <223> x= val, Leu, Ile
<220>
   <221> X
   <222> (66)..(66)
   <223> X= Gly or Ala
<220>
   <221> X
   <222> (67)..(67)
   <223> X= Ala, Gln, Val or Thr
<220>
   <221> X
   <222> (68)..(68)
   <223> X= Val, Met, Leu
<220>
   <221> X
   <222> (70)..(70)
   <223> x= Ser, Asp, Gly
<220>
   <221> X
   <222> (72)..(72)
   <223> X= Thr, Asn, Ser
<220>
   <221> X
   <222> (73)..(73)
   <223> x= Trp, Tyr or no amino acid
<220>
   <221> X
   <222> (74)..(74)
   <223> x= Thr, Val or Gly
<220>
   <221> X
   <222> (75)..(75)
   <223> X= Pro, Thr, Lys or Ser
<220>
   <221> X
   <222> (76)..(76)
   <223> X= Gly, Trp or Asp
<220>
   <221> X
   <222> (77)..(77)
   <223> X= Asp, Lys or Gly
<220>
   <221> X
   <222> (78)..(78)
   <223> x= Gly, Glu, Asp, val or no amino acid
<220>
   <221> X
   <222> (79)..(79)
   <223> X= Leu, val, Lys or Thr
<220>
   <221> X
   <222> (80)..(80)
   <223> X= Thr, Ile, Gly or Leu
<220>
   <221> X
   <222> (81)..(81)
   <223> X= Gly, Asn or Phe
<220>
   <221> X
   <222> (82)..(82)
   <223> X= Phe, Tyr, Ile or Gly
<220>
   <221> X
   <222> (83)..(83)
   <223> X= Gly, Ser, Glu or Asp
<220>
   <221> X
   <222> (84)..(84)
   <223> X= Gly or no amino acid
<220>
   <221> X
   <222> (85)..(85)
   <223> x= Gln or no amino acid
<220>
   <221> X
   <222> (86)..(86)
   <223> x= Phe or no amino acid
<220>
   <221> X
   <222> (87)..(87)
   <223> X= Ser, Val or no amino acid
<220>
   <221> X
   <222> (88)..(88)
   <223> X= Lys, Thr or no amino acid
<220>
   <221> X
   <222> (89)..(89)
   <223> X= Ile, Asn or no amino acid
<220>
   <221> X
   <222> (90)..(90)
   <223> X= Trp, Leu or no amino acid
<220>
   <221> X
   <222> (91)..(91)
   <223> X= Lys, Asp, Glu or Asn
<220>
   <221> X
   <222> (92)..(92)
   <223> X= Asp, Lys, Asn or Gly
<220>
   <221> X
   <222> (93)..(93)
   <223> X= Asn, Ala, Leu, Ser or Thr
<220>
   <221> X
   <222> (94)..(94)
   <223> X= Tyr, Phe or Ile
<220>
   <221> X
   <222> (95)..(95)
   <223> X= Thr, Asn, Gly or Ser
<220>
   <221> X
   <222> (96)..(96)
   <223> X= Asp, Thr, Gln or no amino acid
<220>
   <221> X
   <222> (97)..(97)
   <223> X= Asn or no amino acid
<220>
   <221> X
   <222> (98)..(98)
   <223> x= Val or no amino acid
<220>
   <221> X
   <222> (99)..(99)
   <223> X= Thr, Lys,Phe or no amino acid
<220>
   <221> X
   <222> (100)..(100)
   <223> X= Gly or Asp
<220>
   <221> X
   <222> (101)..(101)
   <223> X= Glu, Tyr, Asn or Leu
<220>
   <221> X
   <222> (102)..(102)
   <223> X= Ser, Gly, Pro, Trp or Tyr
<220>
   <221> X
   <222> (103)..(103)
   <223> X= Thr, Ile or Asn
<220>
   <221> X
   <222> (104)..(104)
   <223> X= Gly, Phe, Glu, Asp or Val
<220>
   <221> X
   <222> (105)..(105)
   <223> X= Ala, Gly, Lys, Asp or Asn
<220>
   <221> X
   <222> (106)..(106)
   <223> X= Gln, Gly, Asn, His or Arg
<220>
   <221> X
   <222> (107)..(107)
   <223> X= Lys, Asn, Thr or Leu
<220>
   <221> X
   <222> (108)..(108)
   <223> X= Phe, Trp, Thr or Pro
<220>
   <221> X
   <222> (109)..(109)
   <223> X= Gly or Arg
<220>
   <221> X
   <222> (110)..(110)
   <223> X= Tyr, Phe, Asp, Thr or Gly
<220>
   <221> X
   <222> (111)..(111)
   <223> X= Gly, Tyr, Asp, Glu or no amino acid
<220>
   <221> X
   <222> (112)..(112)
   <223> X= Ala or Asp
<220>
   <221> X
   <222> (113)..(113)
   <223> X= Leu, Val, Met or Gln
<220>
   <221> X
   <222> (114)..(114)
   <223> X= Gly, Asn or Thr
<220>
   <221> X
   <222> (115)..(115)
   <223> x= val, Ala, or Phe
<220>
   <221> X
   <222> (116)..(116)
   <223> X= Val or Leu or Gly
<220>
   <221> X
   <222> (117)..(117)
   <223> X= Asn or no amino acid
<220>
   <221> X
   <222> (118)..(118)
   <223> x= Ser, Ala, Lys or no amino acid
<220>
   <221> X
   <222> (119)..(119)
   <223> x= Ile, Gly, val or no amino acid
<220>
   <221> x
   <222> (120)..(120)
   <223> x= Leu, Met or no amino acid
<220>
   <221> X
   <222> (121)..(121)
   <223> X= Asn, Gln, Thr or no amino acid
<220>
   <221> X
   <222> (122)..(122)
   <223> x= val, Ile, Thr or no amino acid
<220>
   <221> X
   <222> (123)..(123)
   <223> x= Ala, Leu or no amino acid
<220>
   <221> X
   <222> (125)..(125)
   <223> x= Asn, Gly, Ile, Met or Leu
<220>
   <221> X
   <222> (126)..(126)
   <223> X= Leu or Ala
<220>
   <221> X
   <222> (127)..(127)
   <223> x= Ala or Ser
<220>
   <221> X
   <222> (128)..(128)
   <223> X= Ala or val
<220>
   <221> X
   <222> (129)..(129)
   <223> X= Ile, val or Gly
<220>
   <221> X
   <222> (131)..(131)
   <223> X= Asn or Thr
<220>
   <221> X
   <222> (134)..(134)
   <223> X= Phe, Ser, Val, Thr or Met
<220>
   <221> X
   <222> (135)..(135)
   <223> x= Gly, Ser, Ala, Thr or Lys
<220>
   <221> X
   <222> (136)..(136)
   <223> X= Thr, Ser, Pro or Asp
<220>
   <221> X
   <222> (137)..(137)
   <223> X= Ala, Ile or Thr
<220>
   <221> X
   <222> (138)..(138)
   <223> X= Lys, Gly or Ala
<220>
   <221> X
   <222> (139)..(139)
   <223> X= Asn, Leu or Val
<220>
   <221> X
   <222> (140)..(140)
   <223> X= Ile, Glu, Thr, Gly or Leu
<220>
   <221> X
   <222> (141)..(141)
   <223> X= Val, Thr or Phe
<220>
   <221> X
   <222> (142)..(142)
   <223> X= Gly, Lys, Asn or Ala
<220>
   <221> X
   <222> (143)..(143)
   <223> X= Glu, Ser, Asp or Lys
<220>
   <221> X
   <222> (144)..(144)
   <223> X= Gly, Thr, Lys
<220>
   <221> X
   <222> (145)..(145)
   <223> X= Val, Thr or Glu
<220>
   <221> X
   <222> (146)..(146)
   <223> X= Tyr, Leu, Val or Thr
<220>
   <221> X
   <222> (147)..(147)
   <223> X= Lys, Pro, Gly or Asn
<220>
   <221> X
   <222> (148)..(148)
   <223> X= Ala, Thr, Phe, Ile or Ser
<220>
   <221> X
   <222> (149)..(149)
   <223> X= Leu, Thr, Asn or no amino acid
<220>
   <221> X
   <222> (150)..(150)
   <223> X= val, Gly or no amino acid
<220>
   <221> X
   <222> (151)..(151)
   <223> X= Lys, Gln or no amino acid
<220>
   <221> X
   <222> (152)..(152)
   <223> x= Leu, Val or no amino acid
<220>
   <221> X
   <222> (153)..(153)
   <223> x= Trp or no amino acid
<220>
   <221> X
   <222> (154)..(154)
   <223> X= Gly or no amino acid
<220>
   <221> X
   <222> (155)..(155)
   <223> X= Asp or no amino acid
<220>
   <221> X
   <222> (156)..(156)
   <223> X= Leu or no amino acid
<220>
   <221> X
   <222> (157)..(157)
   <223> X= Pro or no amino acid
<220>
   <221> X
   <222> (158)..(158)
   <223> X= Asn or no amino acid
<220>
   <221> X
   <222> (159)..(159)
   <223> X= Asn or no amino acid
<220>
   <221> X
   <222> (160)..(160)
   <223> X= Gly or no amino acid
<220>
   <221> X
   <222> (161)..(161)
   <223> x= Gly or no amino acid
<220>
   <221> X
   <222> (162)..(162)
   <223> X= Ser or no amino acid
<220>
   <221> X
   <222> (163)..(163)
   <223> X= Gly or no amino acid
<220>
   <221> X
   <222> (164)..(164)
   <223> X= Trp or no amino acid
<220>
   <221> X
   <222> (165)..(165)
   <223> X= Val or no amino acid
<220>
   <221> X
   <222> (166)..(166)
   <223> x= Gly or no amino acid
<400> 1
   Met Met Met Pro Ala Asn Xaa Xaa Xaa Xaa Xaa Xaa Xaa Xaa Xaa Xaa
   1               5                   10                  15
   Xaa Val Xaa Gly Gly Xaa Xaa Xaa Xaa Xaa Xaa Xaa Xaa Xaa Xaa Xaa
               20                  25                  30
   Xaa Xaa Xaa Xaa Xaa Xaa Xaa Xaa Xaa Xaa Xaa Xaa Xaa Xaa Asn Xaa
           35                  40                  45
   Xaa Xaa Xaa Xaa Xaa Asn Xaa Xaa Xaa Xaa Xaa Xaa Xaa Xaa Xaa Xaa
       50                  55                  60
   Xaa Xaa Xaa Xaa Phe Xaa Gly Xaa Xaa Xaa Xaa Xaa Xaa Xaa Xaa Xaa
   65                  70                  75                  80
   Xaa Xaa Xaa Xaa Xaa Xaa Xaa Xaa Xaa Xaa Xaa Xaa Xaa Xaa Xaa Xaa
                   85                  90                  95
   Xaa Xaa Xaa Xaa Xaa Xaa Xaa Xaa Xaa Xaa Xaa Xaa Xaa Xaa Xaa Xaa
               100                 105                 110
   Xaa Xaa Xaa Xaa Xaa Xaa Xaa Xaa Xaa Xaa Xaa Gly Xaa Xaa Xaa Xaa
           115                 120                 125
   Xaa Tyr Xaa Leu Gly Xaa Xaa Xaa Xaa Xaa Xaa Xaa Xaa Xaa Xaa Xaa
       130                 135                 140
   Xaa Xaa Xaa Xaa Xaa Xaa Xaa Xaa Xaa Xaa Xaa Xaa Xaa Xaa Xaa Xaa
   145                 150                 155                 160
   Xaa Xaa Xaa Xaa Xaa Xaa
                   165
<210> 2
   <211> 135
   <212> PRT
   <213> Faecalibacterium prausnitzii
<400> 2
   Met Met Met Pro Ala Asn Tyr Ser Val Ile Ala Glu Asn Glu Met Thr
   1 5 10 15
   Tyr Val Asn Gly Gly Ala Asn Phe Ile Asp Ala Ile Gly Ala Val Thr
   20 25 30
   Ala Pro Ile Trp Thr Leu Asp Asn Val Lys Thr Phe Asn Thr Asn Ile
   35 40 45
   Val Thr Leu Val Gly Asn Thr Phe Leu Gln Ser Thr Ile Asn Arg Thr
   50 55 60
   Ile Gly Val Leu Phe Ser Gly Asn Thr Thr Trp Lys Glu Val Gly Asn
   65 70 75 80
   Ile Gly Lys Asn Leu Phe Gly Thr Asn Val Lys Gly Asn Pro Ile Glu
   85 90 95
   Lys Asn Asn Phe Gly Asp Tyr Ala Met Asn Ala Leu Gly Ile Ala Ala
   100 105 110
   Ala Val Tyr Asn Leu Gly Val Ala Pro Thr Lys Asn Thr Val Lys Glu
   115 120 125
   Thr Glu Val Lys Phe Thr Val
   130 135
<210> 3
   <211> 143
   <212> PRT
   <213> Faecalibacterium prausnitzii
<400> 3
   Met Met Met Pro Ala Asn Phe Ser Ala Val Ser Glu Asn Glu Met Thr
   1 5 10 15
   Tyr Val Met Gly Gly Ser Val Ala Asp Tyr Leu Ala Pro Ala Met Gly
   20 25 30
   Ala Ala Gln Trp Gln Asn Phe His Lys Asn Leu Ile Thr Ile Val Gly
   35 40 45
   Asn Lys Tyr Val Gln Gly Phe Leu Asp Asn Thr Val Gly Ala Val Phe
   50 55 60
   Ser Gly Thr Trp Thr Pro Gly Asp Gly Leu Thr Gly Phe Gly Gly Gln
   65 70 75 80
   Phe Ser Lys Ile Trp Lys Asp Asn Tyr Thr Asp Asn Val Thr Gly Glu
   85 90 95
   Ser Thr Gly Ala Gln Lys Phe Gly Tyr Gly Ala Leu Gly Val Val Asn
   100 105 110
   Ser Ile Leu Asn Val Ala Gly Asn Leu Ala Ala Ile Tyr Asn Leu Gly
   115 120 125
   Phe Gly Thr Ala Lys Asn Ile Val Gly Glu Gly Val Tyr Lys Ala
   130 135 140
<210> 4
   <211> 143
   <212> PRT
   <213> Faecalibacterium prausnitzii
<400> 4
   Met Met Met Pro Ala Asn Phe Ser Ala Val Ser Glu Asn Glu Met Thr
   1 5 10 15
   Tyr Val Met Gly Gly Ser Val Ala Asp Tyr Leu Ala Pro Ala Met Gly
   20 25 30
   Ala Ala Gln Trp Gln Asn Phe His Lys Asn Leu Ile Thr Ile Val Gly
   35 40 45
   Asn Lys Tyr Val Gln Gly Phe Leu Asp Asn Thr Val Gly Ala Met Phe
   50 55 60
   Ser Gly Thr Trp Thr Pro Gly Asp Gly Leu Thr Gly Phe Gly Gly Gln
   65 70 75 80
   Phe Ser Thr Ile Trp Lys Lys Asn Tyr Thr Asp Asn Val Thr Asp Glu
   85 90 95
   Ser Thr Gly Ala Gln Lys Phe Gly Tyr Gly Ala Leu Gly Val Val Asn
   100 105 110
   Ser Ile Leu Asn Val Ala Gly Asn Leu Ala Ala Ile Tyr Asn Leu Gly
   115 120 125
   Phe Gly Thr Ala Lys Asn Ile Val Gly Glu Gly Val Tyr Lys Ala
   130 135 140
<210> 5
   <211> 136
   <212> PRT
   <213> Faecalibacterium prausnitzii
<400> 5
   Met Met Met Pro Ala Asn Phe Ser Ala Val Ala Glu Asn Glu Met Thr
   1 5 10 15
   Tyr Val Val Gly Gly Ser Leu Val Asp Val Leu Ala Pro Ala Met Thr
   20 25 30
   Thr Ala Asn Trp Gln Asn Val Ser Ala Asn Val Ile Lys Ile Val Gly
   35 40 45
   Asn Ser Phe Leu Ala Lys Tyr Thr Asn Asp Val Leu Ala Gln Leu Phe
   50 55 60
   Asp Gly Asn Tyr Val Pro Gly Asp Val Ile Gly Tyr Ser Val Lys Asn
   65 70 75 80
   Leu Asp Lys Ala Tyr Asn Lys Gly Tyr Gly Thr Phe Gly Gly Asn Trp
   85 90 95
   Gly Phe Ala Val Gly Ala Leu Asn Ala Gly Met Gln Ile Leu Gly Gly
   100 105 110
   Leu Ser Ala Ile Tyr Thr Leu Gly Ser Ser Ser Ile Gly Leu Glu Thr
   115 120 125
   Lys Ser Gly Thr Leu Pro Thr Leu
   130 135
<210> 6
   <211> 142
   <212> PRT
   <213> Faecalibacterium prausnitzii
<400> 6
   Met Met Met Pro Ala Asn Phe Thr Ala Val Asn Ser Glu Val Val Tyr
   1 5 10 15
   Gly Gly Ala Asp Leu Phe Thr Ile Leu Ala Asp Thr Thr Ala Pro Ile
   20 25 30
   Trp Asn Ala Ala Asn Val Lys Lys Phe Asn Thr Asn Leu Ile Thr Leu
   35 40 45
   Ile Ser Asn Ser Phe Phe Lys Lys Thr Val Ser Asn Thr Leu Gly Val
   50 55 60
   Met Phe Gly Gly Asn Trp Gly Lys Asp Gly Asp Lys Ile Phe Gly Glu
   65 70 75 80
   Glu Gly Ser Ile Asn Gln Asn Val Phe Gly Leu Trp Asn Asp Asp His
   85 90 95
   Thr Thr Arg Thr Asp Asp Met Thr Phe Gly Asn Lys Val Met Gln Val
   100 105 110
   Leu Gly Met Ala Ala Val Gly Tyr Thr Leu Gly Thr Thr Asp Ala Lys
   115 120 125
   Val Gly Phe Asn Asp Gly Val Tyr Gly Ile Asn Gly Lys Leu
   130 135 140
<210> 7
   <211> 147
   <212> PRT
   <213> Faecalibacterium prausnitzii
<400> 7
   Met Met Met Pro Ala Asn Phe Ser Ala Val Asn Ala Glu Val Val Tyr
   1               5                   10                  15
   Gly Gly Ala Val Ala Asp Tyr Leu Pro Ser Ala Trp Thr Ala Glu Ser
               20                  25                  30
   Val Lys Arg Phe Asn Ser Asn Ile Ile Thr Leu Val Ser Asn Ser Phe
           35                  40                  45
   Thr Ser His Leu Leu Lys Ala Thr Leu Gly Thr Met Phe Ser Gly Ser
       50                  55                  60
   Trp Gly Ser Asp Gly Val Thr Leu Phe Gly Asp Asn Gly Thr Phe Ser
   65                  70                  75                  80
   Gly Leu Tyr Asn Val Asn Arg Leu Pro Gly Gly Glu Ala Gln Thr Phe
                   85                  90                  95
   Gly Asn Lys Ile Met Thr Thr Leu Gly Leu Ala Ser Val Val Tyr Thr
               100                 105                 110
   Leu Gly Met Lys Asp Ala Ala Val Leu Thr Ala Lys Lys Val Thr Asn
           115                 120                 125
   Ser Asn Gly Gln Val Trp Gly Asp Leu Pro Asn Asn Gly Gly Ser Gly
       130                 135                 140
   Trp Val Gly
   145
<210> 8
   <211> 71
   <212> PRT
   <213> Artificial Sequence
<220>
   <223> consensus sequence
<220>
   <221> x
   <222> (7)..(7)
   <223> X= Phe or Tyr
<220>
   <221> X
   <222> (8)..(8)
   <223> X= Ser or Thr
<220>
   <221> x
   <222> (9)..(9)
   <223> X= Ala or Val
<220>
   <221> X
   <222> (10)..(10)
   <223> X= Val or Ile
<220>
   <221> X
   <222> (11)..(11)
   <223> X= Ser, Ala or Asn
<220>
   <221> X
   <222> (12)..(12)
   <223> X= Glu or no amino acid
<220>
   <221> X
   <222> (13)..(13)
   <223> x= Asn or no amino acid
<220>
   <221> X
   <222> (14)..(14)
   <223> X= Glu or no amino acid
<220>
   <221> X
   <222> (15)..(15)
   <223> X= Met, Ser or Ala
<220>
   <221> X
   <222> (16)..(16)
   <223> X= Thr or Glu
<220>
   <221> X
   <222> (17)..(17)
   <223> X= Tyr or val
<220>
   <221> X
   <222> (19)..(19)
   <223> X= Met, val, Asn or Tyr
<220>
   <221> X
   <222> (22)..(22)
   <223> X= Ser or Ala
<220>
   <221> X
   <222> (23)..(23)
   <223> x= Asn, Asp or no amino acid
<220>
   <221> X
   <222> (24)..(24)
   <223> X= Phe, Leu or no amino acid
<220>
   <221> x
   <222> (25)..(25)
   <223> X= Ile, Phe or no amino acid
<220>
   <221> x
   <222> (26)..(26)
   <223> X= Asp, Thr or no amino acid
<220>
   <221> X
   <222> (27)..(27)
   <223> X= Ala, Ile or no amino acid
<220>
   <221> X
   <222> (28)..(28)
   <223> X= val, Leu or Ile
<220>
   <221> X
   <222> (29)..(29)
   <223> X= Ala, val or Gly
<220>
   <221> X
   <222> (30)..(30)
   <223> X= Ala or Asp
<220>
   <221> X
   <222> (31)..(31)
   <223> X= Tyr, val or Thr
<220>
   <221> X
   <222> (32)..(32)
   <223> X= Leu or Thr
<220>
   <221> X
   <222> (33)..(33)
   <223> X= Ala or Pro
<220>
   <221> X
   <222> (34)..(34)
   <223> X= Pro or Ser
<220>
   <221> X
   <222> (35)..(35)
   <223> X= Ala or Ile
<220>
   <221> X
   <222> (36)..(36)
   <223> X= Met or Trp
<220>
   <221> X
   <222> (37)..(37)
   <223> X= Gly, Thr or Asn
<220>
   <221> X
   <222> (38)..(38)
   <223> X= Ala, Thr or Leu
<220>
   <221> X
   <222> (39)..(39)
   <223> X= Ala, Asp or Glu
<220>
   <221> X
   <222> (40)..(40)
   <223> X= Gln, Asn or Ser
<220>
   <221> X
   <222> (41)..(41)
   <223> X= Trp or val
<220>
   <221> X
   <222> (42)..(42)
   <223> X= Gln or Lys
<220>
   <221> X
   <222> (43)..(43)
   <223> X= Asn, Thr, Lys or Arg
<220>
   <221> X
   <222> (44)..(44)
   <223> X= Phe or val
<220>
   <221> X
   <222> (45)..(45)
   <223> X= His, Ser, Asn
<220>
   <221> X
   <222> (46)..(46)
   <223> X= Lys, Ala, Thr or Ser
<220>
   <221> X
   <222> (48)..(48)
   <223> X= Leu, Val or Ile
<220>
   <221> X
   <222> (49)..(49)
   <223> X= Ile or val
<220>
   <221> X
   <222> (50)..(50)
   <223> X= Thr or Lys
<220>
   <221> X
   <222> (51)..(51)
   <223> X= Ile or Leu
<220>
   <221> X
   <222> (52)..(52)
   <223> X= Val or Ile
<220>
   <221> X
   <222> (53)..(53)
   <223> X= Gly or Ser
<220>
   <221> X
   <222> (55)..(55)
   <223> X= Lys, Ser or Thr
<220>
   <221> X
   <222> (56)..(56)
   <223> X= Tyr or Phe
<220>
   <221> X
   <222> (57)..(57)
   <223> X= Val, Leu, Phe or Thr
<220>
   <221> X
   <222> (58)..(58)
   <223> X= Gln, Ala, Lys or Ser
<220>
   <221> X
   <222> (59)..(59)
   <223> X59= Gly, Lys, Ser or His
<220>
   <221> X
   <222> (60)..(60)
   <223> X= Phe, Tyr, Thr or Leu
<220>
   <221> X
   <222> (61)..(61)
   <223> X= Leu, Thr, Ile, or Val
<220>
   <221> X
   <222> (62)..(62)
   <223> X=AS_{P}, Asn, Ser or Lys
<220>
   <221> X
   <222> (63)..(63)
   <223> X= Asn, Asp, Arg or Ala
<220>
   <221> X
   <222> (64)..(64)
   <223> X= Thr or Val
<220>
   <221> X
   <222> (65)..(65)
   <223> X= Val, Leu or Ile
<220>
   <221> X
   <222> (66)..(66)
   <223> X= Gly or Ala
<220>
   <221> X
   <222> (67)..(67)
   <223> X= Ala, Gln, Val or Thr
<220>
   <221> X
   <222> (68)..(68)
   <223> X= val, Met, Leu
<220>
   <221> X
   <222> (70)..(70)
   <223> X= Ser, Asp, Gly
<400> 8
   Met Met Met Pro Ala Asn Xaa Xaa Xaa Xaa Xaa Xaa Xaa Xaa Xaa Xaa
   1               5                   10                  15
   Xaa Val Xaa Gly Gly Xaa Xaa Xaa Xaa Xaa Xaa Xaa Xaa Xaa Xaa Xaa
               20                  25                  30
   Xaa Xaa Xaa Xaa Xaa Xaa Xaa Xaa Xaa Xaa Xaa Xaa Xaa Xaa Asn Xaa
           35                  40                  45
   Xaa Xaa Xaa Xaa Xaa Asn Xaa Xaa Xaa Xaa Xaa Xaa Xaa Xaa Xaa Xaa
       50                  55                  60
   Xaa Xaa Xaa Xaa Phe Xaa Gly
   65                  70
<210> 9
   <211> 10
   <212> PRT
   <213> Artificial Sequence
<220>
   <223> consensus sequence
<220>
   <221> X
   <222> (2)..(2)
   <223> x= Asn, Gly, Ile, Met or Leu
<220>
   <221> X
   <222> (3)..(3)
   <223> X= Leu or Ala
<220>
   <221> X
   <222> (4)..(4)
   <223> X= Ala or Ser
<220>
   <221> X
   <222> (5)..(5)
   <223> X= Ala or val
<220>
   <221> X
   <222> (6)..(6)
   <223> X= Ile, val or Gly
<220>
   <221> X
   <222> (8)..(8)
   <223> X= Asn or Thr
<400> 9
   Gly Xaa Xaa Xaa Xaa Xaa Tyr Xaa Leu Gly
   1               5                   10
<210> 10
   <211> 43
   <212> PRT
   <213> Faecalibacterium prausnitzii
<400> 10
   Phe Ser Gly Asn Thr Thr Trp Lys Glu Val Gly Asn Ile Gly Lys Asn
   1 5 10 15
   Leu Phe Gly Thr Asn Val Lys Gly Asn Pro Ile Glu Lys Asn Asn Phe
   20 25 30
   Gly Asp Tyr Ala Met Asn Ala Leu Gly Ile Ala
   35 40
<210> 11
   <211> 16
   <212> PRT
   <213> Faecalibacterium prausnitzii
<400> 11
   Gly Asn Thr Phe Leu Gln Ser Thr Ile Asn Arg Thr Ile Gly Val Leu
   1 5 10 15
<210> 12
   <211> 17
   <212> PRT
   <213> Faecalibacterium prausnitzii
<400> 12
   Val Gly Asn Thr Phe Leu Gln Ser Thr Ile Asn Arg Thr Ile Gly Val
   1 5 10 15
   Leu
<210> 13
   <211> 18
   <212> PRT
   <213> Faecalibacterium prausnitzii
<400> 13
   Leu Val Gly Asn Thr Phe Leu Gln Ser Thr Ile Asn Arg Thr Ile Gly
   1 5 10 15
   Val Leu
<210> 14
   <211> 19
   <212> PRT
   <213> Faecalibacterium prausnitzii
<400> 14
   Thr Leu Val Gly Asn Thr Phe Leu Gln Ser Thr Ile Asn Arg Thr Ile
   1 5 10 15
   Gly Val Leu
<210> 15
   <211> 20
   <212> PRT
   <213> Faecalibacterium prausnitzii
<400> 15
   Val Thr Leu Val Gly Asn Thr Phe Leu Gln Ser Thr Ile Asn Arg Thr
   1 5 10 15
   Ile Gly Val Leu
   20
<210> 16
   <211> 15
   <212> PRT
   <213> Faecalibacterium prausnitzii
<400> 16
   Asn Thr Phe Leu Gln Ser Thr Ile Asn Arg Thr Ile Gly Val Leu
   1 5 10 15
<210> 17
   <211> 24
   <212> PRT
   <213> Faecalibacterium prausnitzii
<400> 17
   Ala Ala Val Tyr Asn Leu Gly Val Ala Pro Thr Lys Asn Thr Val Lys
   1 5 10 15
   Glu Thr Glu Val Lys Phe Thr Val
   20
<210> 18
   <211> 47
   <212> PRT
   <213> Faecalibacterium prausnitzii
<400> 18
   Asn Tyr Ser Val Ile Ala Glu Asn Glu Met Thr Tyr Val Asn Gly Gly
   1               5                   10                  15
   Ala Asn Phe Ile Asp Ala Ile Gly Ala Val Thr Ala Pro Ile Trp Thr
               20                  25                  30
   Leu Asp Asn Val Lys Thr Phe Asn Thr Asn Ile Val Thr Leu Val
           35                  40                  45
<210> 19
   <211> 408
   <212> DNA
   <213> Faecalibacterium prausnitzii
<400> 19
   atgatgatgc ctgcaaacta ctctgttatc gcagagaacg aaatgaccta cgtcaacggt      60
   ggcgctaact tcatcgacgc tatcggcgct gttaccgctc ctatctggac tctggacaac     120
   gttaagacct tcaacaccaa catcgtgact ctggttggca acaccttcct gcagtccacc     180
   attaaccgca ccatcggtgt cctgttcagc ggcaacacca cctggaagga agtcggcaac     240
   atcggcaaga acctgttcgg caccaatgtt aagggcaacc cgatcgagaa gaacaacttt     300
   ggtgactatg ctatgaacgc tctgggcatt gctgctgctg tctacaacct gggcgtggct     360
   cccaccaaga acaccgtcaa ggagactgag gttaagttca ctgtctaa                  408
<210> 20
   <211> 17
   <212> PRT
   <213> Faecalibacterium prausnitzii
<400> 20
   Lys Gly Asn Thr Phe Leu Gln Ser Thr Ile Asn Arg Thr Ile Gly Val
   1               5                   10                  15
   Leu
<210> 21
   <211> 46
   <212> PRT
   <213> Faecalibacterium prausnitzii
<400> 21
   Val Lys Gly Asn Pro Ile Glu Lys Asn Asn Phe Gly Asp Tyr Ala Met
   1               5                   10                  15
   Asn Ala Leu Gly Ile Ala Ala Ala Val Tyr Asn Leu Gly Val Ala Pro
               20                  25                  30
   Thr Lys Asn Thr Val Lys Glu Thr Glu Val Lys Phe Thr Val
           35                  40                  45
<210> 22
   <211> 8
   <212> PRT
   <213> Artificial Sequence
<220>
   <223> signal peptide
<400> 22
   Leu Glu Ile Ser Ser Thr Cys Asp
   1               5

## Description

### FIELD OF THE INVENTION

The present invention relates to the treatment of inflammatory diseases based on the discovery of a new anti-inflammatory protein.

### BACKGROUND OF THE INVENTION

Inflammation is a natural biological process, which constitutes a normal part of the response to injuries or infections. This process contributes to the protection of the organism against intern or extern aggressions. However, a dysfunction of the inflammation mechanisms, particularly a persistent or too abundant inflammation may cause important painful and life threatening diseases. Such diseases comprise skin disorders, bowel disorders, some neurological disorders, arthritis, autoimmune diseases... Several of these inflammatory diseases remain without treatment or without sufficient treatment. Thus, studying and finding new anti-inflammatory treatment strategies constitutes a major matter in medicine and biomedical research.

Inflammatory bowel disease is a group of disorders characterized by a chronic and relapsing inflammation of the gastrointestinal tract. The most common form of this group is Crohn disease. The pathogenesis involves an inappropriate and ongoing activation of the mucosal immune system driven by the presence of the intestinal microbiota in a genetically predisposed patient.

*Faecalibacterium prausnitzii,* a strictly anaerobic commensal bacterium, has been shown to be decreased in patients with ileal Crohn disease both in faeces and in the mucosal compartment. Its decrease is associated with an early recurrence of the disease in the model of postoperative recurrence after ileo-caecal resection. The culture supernatant of *F. prausnitzii* exerts an anti-inflammatory activity in both *in vitro* and *in vivo* murine models of TNBS-induced colitis.

### SUMMARY OF THE INVENTION

The present invention is based on the discovery by the present inventors of the anti-inflammatory properties of a protein (and of six derived fragments thereof) of the bacterium *Faecalibacterium prausnitzii.*

The present invention relates to a polypeptide comprising or consisting of the amino acid sequence SEQ ID NO:1, for use in the treatment or prevention of an inflammatory disease.

Particularly, said inflammatory disease is a bowel inflammatory disease. Preferably, said inflammatory disease is Crohn disease.

The invention further encompasses a nucleic acid sequence encoding a polypeptide of the invention, a vector comprising a nucleic acid of the invention and a host cell comprising a nucleic acid sequence and/or a vector of the invention, for use in the treatment or prevention of an inflammatory disease.

The invention also concerns a pharmaceutical composition comprising a polypeptide, a nucleic acid sequence, a vector or a host cell of the invention and a pharmaceutically acceptable carrier.

Finally, the invention still concerns a pharmaceutical composition comprising a polypeptide, a nucleic acid sequence, a vector or a host cell of the invention and a pharmaceutically acceptable carrier for use in the treatment of inflammatory disease.

### BRIEF DESCRIPTION OF THE DRAWINGS

**Figure 1** shows effect of protein ZP05614546.1 on Carma-1-dependant NF-kB activation using HEK293T cells and NPκB luciferase reporter.
   *MAM= MAM plasmid transfection comprising protein ZP05614546.1; EMAM* = *empty MAM plasmid transfection*
   *Ecarma1 : empty Carma 1 plasmid tranfection.*
**Figure 2** shows effect of protein ZP05614546.1 on LPS-dependent NPκB activation using HEK293T cells stably expressing TLR4, MD2 and CD14 and NPκB luciferase reporter.
   *MAM= MAM plasmid transfection comprising protein ZP05614546.1; EMAM* = *empty MAM plasmid transfection.*
**Figure 3** shows alignments of homolog sequences of the protein of the invention.
   * *: Amino acid identity,* : *: Amino acid high homology,* . *: Amino acid homology.*
**Figure 4** shows an anti-inflammatory effect of peptides defined by SEQ ID NO:20-21 on HT29-MTX cells, by an assessment of IL8 production after stimulation of HT29-MTX cells incubated or not with said peptides and stimulated by TNFα.

### DETAILED DESCRIPTION OF THE INVENTION

### Polypeptides of the invention

A first aspect of the invention relates to a polypeptide comprising or consisting of the amino acid sequence SEQ ID NO:1 for use in the treatment or prevention of an inflammatory disease.

The term "inflammatory disease" has its general meaning in the art and refers to any disease and condition associated with inflammation. The term may include, but is not limited to, (1) inflammatory or allergic diseases such as systemic anaphylaxis or hypersensitivity responses, drug allergies, insect sting allergies; inflammatory bowel diseases, such as Crohn disease, ulcerative colitis, ileitis and enteritis; vaginitis; psoriasis and inflammatory dermatoses such as dermatitis, eczema, atopic dermatitis, allergic contact dermatitis, urticaria; vasculitis; spondyloarthropathies; scleroderma; respiratory allergic diseases such as asthma, allergic rhinitis, hypersensitivity lung diseases, and the like, (2) autoimmune diseases, such as arthritis (rheumatoid and psoriatic), osteoarthritis, multiple sclerosis, systemic lupus erythematosus, diabetes mellitus, glomerulonephritis, and the like, (3) graft rejection (including allograft rejection and graft-v- host disease), and (4) other diseases in which undesired inflammatory responses are to be inhibited (e. g., atherosclerosis, myositis, inflammatory CNS disorders such as stroke and closed-head injuries, neurodegenerative diseases, Alzheimer's disease, encephalitis, meningitis, osteoporosis, gout, hepatitis, nephritis, sepsis, sarcoidosis, conjunctivitis, otitis, chronic obstructive pulmonary disease, sinusitis and Bechet's syndrome).

In a particular embodiment of the invention, said inflammatory disease is an inflammatory bowel disease, comprising Crohn disease, ulcerative colitis, ileitis and enteritis.

In a preferred embodiment of the invention, said inflammatory disease is Crohn disease.

In the context of the invention, the term "treating" or "treatment" means reversing, alleviating, inhibiting the progress of, or preventing the disorder or condition to which such term applies, or one or more symptoms of such disorder or condition. The term "preventing" or "prevention" refers to preventing the disease or condition from occurring in a subject which has not yet been diagnosed as having it.

In another particular embodiment, said inflammatory disease is an inflammatory disease resulting from an activation of the NPκB pathway.

The polypeptide of the invention comprises or consists of the amino acid sequence SEQ ID NO:1. Said sequence corresponds to a consensus sequence between at least five orthologs protein sequences.

| | |
|---|---|
| SEQ ID NO:1 | |
| | |
| | Wherein X1= F,Y; X2= S,T; X3=A,V; X4=V,I; X5=S,A,N; X6=E, -; X7=N, - ; X8=E, - ; X9=M,S,A; X10=T,E ; X11=Y,V; X12=M,V,N,Y; X13=S,A; X14=N,D, -; X15=F,L, -; X16=I,F, -; X17=D,T, -; X18=A,I, -; X19=V,L,I; X20=A,V,G; X21=A,D; X22=Y,V,T; X23=L,T; X24=A,P; X25=P,S ; X26=A,I; X27=M,W; X28=G,T,N ; X29=A,T,L; X30=A,D,E; X31=Q,N,S; X32=W,V ; X33=Q,K ; X34=N,T,K,R ; X35=F,V; X36=H,S,N; X37=K,A,T,S; X38=L,V,I; X39=I,V; X40=T,K; X41=I,L; X42=V,I; X43=G,S; X44=K,S,T; X45=Y,F; X46=V,L,F,T; X47=Q,A,K,S; X48=G,K,S,H; X49=F,Y,T,L; X50=L,T,I,V; X51=D,N,S,K; X52=N,D,R,A; X53=T,V; X54=V,L,I; X55=G,A; X56=A,Q,V,T; X57=V,M,L; X58=S,D,G; X59=T,N,S; X60=W,Y, -, X61=T,V,G, X62=P,T,K,S, X63=G,W,D, X64=D,K,G, X65=G,E,D,V, -, X66=L,V,K,T, X67=T,I,G,L, X68=G,N,F, ;X69=F,Y,I,G; X70=G,S,E,D; X71=G, -; X72=Q, -; X73=F, -; X74=S,V, -; X75=K,T, -; X76=I,N, -; X77=W,L, -; X78=K,D,E,N; X79=D,K,N,G; X80=N,A,L,S,T; X81=Y,F,I; X82=T,N,G,S; X83=D,T,Q, -; X84=N, -; X85=V, -; X86=T,K,F, -; X87=G,D; X88=E,Y,N,L; X89=S,G,P,W,Y; X90=T,I,N; X91=G,F,E,D,V; X92=A,G,K,D,N; X93=Q,G,N,H,R ;X94=K,N,T,L ; X95=F,W,T,P ;X96=G,R; X97=Y,F,D,T,G; X98=G,Y,D,E,-; X99=A,D; X100=L,V,M,Q; X101=G,N,T; X102=V,A,F; X103=V,L,G ; X104=N,-; X105=S,A,K, -; X106=I,G,V,-; X107=L,M,-; X108=N,Q,T,-; X109=V,I,T,- ; X110=A,L,-; X111=N,G,I,M,L; X112=L,A; X113=A,S; X114=A,V; X115=I,V,G; X116=N,T; X117=F,S,V,T,M; X118=G,S,A,T,K ; X119=T,S,P,D; X120=A,I,T; X121=K,G,A; X122=N,L,V; X123=I,E,T,G,L; X124=V,T,F; X125=G,K,N,A; X126=E,S,D,K; X127=G,T,K; X128=V,T,E,V; X129=Y,L,V,T; X130=K,P,G,N; X131=A,T,FI,S; X132=L,T,N,-; X133=V,G,-; X134=K,Q,-; X135=L,V,-;X136=W,- ; X137=G,-; X138=D,-; X139=L,-; X140=P,-; X141=N,-; X142=N,-; X143=G,-; X144=G,-; X145=S,-; X146=G,-; X147=W,-; X148=V,-; X149=G,-; (- : no amino acid). |

Advantageously, the polypeptides of the invention is selected in the group comprising SEQ ID NO:2, SEQ ID NO:3, SEQ ID NO:4, SEQ ID NO:5, SEQ ID NO:6 and SEQ ID NO:7.

In a particular embodiment, said polypeptide of the invention comprises or consists of the amino acid sequence SEQ ID NO:2.

| | |
|---|---|
| SEQ ID NO:2 | |

In another particular embodiment, said polypeptide of the invention comprises or consists of the amino acid sequence SEQ ID NO:3.

| | |
|---|---|
| SEQ ID NO:3 | |

In another particular embodiment, said polypeptide of the invention comprises or consists of the amino acid sequence SEQ ID NO:4.

| | |
|---|---|
| SEQ ID NO:4 | |

In another particular embodiment, said polypeptide of the invention comprises or consists of the amino acid sequence SEQ ID NO:5.

| | |
|---|---|
| SEQ ID NO:5 | |

In another particular embodiment, said polypeptide of the invention comprises or consists of the amino acid sequence SEQ ID NO:6.

| | |
|---|---|
| SEQ ID NO:6 | |

In another particular embodiment, said polypeptide of the invention comprises or consists of the amino acid sequence SEQ ID NO:7.

| | |
|---|---|
| SEQ ID NO:7 | |

As used herein, the polypeptide of the invention encompasses derivatives or fragments thereof.

According to the invention, the term "derivative thereof" has its general meaning in the art and corresponds to an amino acid sequence or a nucleic acid sequence having at least 90% sequence identity to the referred amino acid sequence or nucleic acid sequence respectively, particularly 95%, and preferably 99%. The term "percentage of identity between two amino acid sequences" or "percentage of identity between two nucleic sequences" refers to the percentage of identic nucleotides or amino acids between two compared sequences, said percentage being obtained with the best alignment of the whole sequence. The term "best alignment" means the alignment that permits to obtain the most elevated identity percentage. It can be realized by using various algorithms and methods well known in the art and computer programs based on said algorithms (GAP, BESTFIT, BLAST P, BLAST N, FASTA, TFASTA, Genetics Computer Group, 575 Science Dr., Madison, WI USA). Preferably, the BLAST algorithm is used.

According to the invention, the term "fragment" refers to a polypeptide being a part of an amino acid sequence of interest and having a length of at least 10 amino acids, particularly at least 15 amino acids, more particularly at least 20 amino acids, preferably at least 30 amino acids, more preferably at least 40 amino acids. Preferably, said fragment has a length of less than 120 amino acids, particularly less than 110 amino acids, preferably less than 100 amino acids. The term is transposable to fragments of nucleic acid sequences.

According to the invention, said derivative and/or fragment of a polypeptide of the invention are conservative derivative or conservative fragments thereof.

By "conservative fragments" and "conservative derivatives" of a polypeptide of the invention, it is respectively meant fragments and derivatives which retain the function, namely the anti-inflammatory properties, of said a polypeptide of the invention. More specifically, a fragment or a derivatives inhibiting the secretion of IL-8 by HT29-MTX cells from at least 50% at a concentration of 100µM or less. Such conservative fragments and conservative derivatives are functional equivalents of said polypeptide. They are "conservative" because they retain the biological function of the original polypeptide, more particularly because they retain an equivalent anti-inflammatory effect.

In a particular embodiment, said conservative fragment of the invention comprises or consists of a polypeptide chosen among SEQ ID NO:8-18.

| | |
|---|---|
| SEQ ID NO:8 | |
| | Wherein X1= F,Y; X2= S,T; X3=A,V; X4=V,I; X5=S,A,N; X6=E, -; X7=N, -; X8=E, -; X9=M,S,A; X10=T,E; X11=Y,V; X12=M,V,N,Y; X13=S,A; X14=N,D, -; X15=F,L, -; X16=I,F, -; X17=D,T, -; X18=A,I, -; X19=V,L,I; X20=A,V,G; X21=A,D; X22=Y,V,T ; X23=L,T ; X24=A,P ; X25=P,S ; X26=A,I; X27=M,W; X28=G,T,N; X29=A,T,L; X30=A,D,E; X31=Q,N,S; X32=W,V ; X33=Q,K ; X34=N,T,K,R ; X35=F,V; X36=H,S,N; X37=K,A,T,S; X38=L,V,I; X39=I,V; X40=T,K; X41=I,L; X42=V,I; X43=G,S; X44=K,S,T; X45=Y,F; X46=V,L,F,T; X47=Q,A,K,S; X48=G,K,S,H; X49=F,Y,T,L; X50=L,T,I,V; X51=D,N,S,K; X52=N,D,R,A; X53=T,V; X54=V,L,I ; X55=G,A ; X56=A,Q,V,T; X57=V,M,L; X58=S,D,G; |
| SEQ ID NO:9 | GX111X112X113 X114X115 YX116LG Wherein X111=N,G,I,M,L; X112=L,A; X113=A,S; X114=A,V; X115=I,V,G; X116=N,T |
| SEQ ID NO:10 | FS GNTTWKEVGN IGKNLFGTNVKGNPIEKNNFGDYAMNALGIA |
| SEQ ID NO:11 | GNTFLQSTINRTIGVL |
| SEQ ID NO:12 | VGNTFLQSTINRTIGVL |
| SEQ ID NO:13 | LVGNTFLQSTINRTIGVL |
| SEQ ID NO:14 | TLVGNTFLQSTINRTIGVL |
| SEQ ID NO:15 | VTLVGNTFLQSTINRTIGVL |
| SEQ ID NO:16 | NTFLQSTINRTIGVL |
| SEQ ID NO:17 | AAVYNLGVAPTKNTVKETEVKFTV |
| SEQ ID NO:18 | NYSVIAENEMTYVNGGANFIDAIGAVTAPIWTLDNVKTFNTNIVTLV |

In another particular embodiment, the polypeptide of the invention corresponds to an amino acid sequence having in the N-term to C-term orientation:
- the sequence SEQ ID NO:8; and
- the sequence SEQ ID NO:9,
wherein the sequence SEQ ID NO:8 and SEQ ID NO:9 are preferably spaced from 80 to 10 amino acids, preferably from 60 to 15 amino acids, and more preferably from 40 to 20 amino acids.

In a preferred embodiment, a polypeptide of the invention or a derivative or a fragment thereof is isolated.

As used herein, the term polypeptide encompasses polypeptides or proteins following post-translational modifications such as glycosylation, phosphorylation or other modifications of some amino acid residues.

The present invention thus relates to a polypeptide as described for use as an anti-inflammatory drug.

As used herein, the term "anti-inflammatory drug" refers to a drug that directly or indirectly reduces inflammation in a tissue.

A polypeptide of the invention may be produced by conventional automated peptide synthesis methods or by recombinant expression. General principles for designing and making peptides and proteins are well known to those of skill in the art.

A polypeptide of the invention may be synthesized in solution or on a solid support in accordance with conventional techniques. Various automatic synthesizers are commercially available and can be used in accordance with known protocols. A polypeptide of the invention may also be synthesized by solid-phase technology employing an exemplary peptide synthesizer such as a MODEL 433A from APPLIED BIOSYSTEMS INC. The purity of any given protein; generated through automated peptide synthesis or through recombinant methods may be determined using reverse phase HPLC analysis. Chemical authenticity of each peptide may be established by any method well known to those of skill in the art.

As an alternative to automated peptide synthesis, recombinant DNA technology may be employed wherein a nucleotide sequence which encodes a protein of choice is inserted into an expression vector, transformed or transfected into an appropriate host cell and cultivated under conditions suitable for expression as described herein below. Recombinant methods are especially preferred for producing longer polypeptides.

A variety of expression vector/host systems may be utilized to contain and express the peptide or protein coding sequence. These include but are not limited to microorganisms such as bacteria transformed with recombinant bacteriophage, plasmid or cosmid DNA expression vectors; yeast transformed with yeast expression vectors; insect cell systems infected with virus expression vectors (e.g., baculovirus); plant cell systems transfected with virus expression vectors (e.g., cauliflower mosaic virus, CaMV; tobacco mosaic virus, TMV) or transformed with bacterial expression vectors (e.g., Ti or pBR322 plasmid); or animal cell systems, including mammalian cell systems. Those of skill in the art are aware of various techniques for optimizing mammalian expression of proteins. Mammalian cells that are useful in recombinant protein productions include, but are not limited to, VERO cells, HeLa cells, Chinese hamster ovary (CHO) cell lines, COS cells (such as COS-7), W138, BHK, HepG2, Caco-2, HT29, HEK, HCT 116, 3T3, RIN, MDCK, A549, PC12, K562 and 293 cells.

Exemplary protocols for the recombinant expression of the peptide substrates or fusion polypeptides in bacteria, yeast and other invertebrates are known to those of skill in the art and a briefly described herein below.

In the recombinant production of the polypeptide of the invention, it would be necessary to employ vectors comprising a nucleic acid sequence encoding such a polypeptide. Methods of preparing such vectors as well as producing host cells transformed with such vectors are well known to those skilled in the art. The polynucleotide molecules used in such an endeavor may be joined to a vector, which generally includes a selectable marker and an origin of replication, for propagation in a host. These elements of the expression constructs are well known to those of skill in the art. Generally, the expression vectors include DNA encoding the given protein being operably linked to suitable transcriptional or translational regulatory sequences, such as those derived from a mammalian, microbial, viral, or insect genes. Examples of regulatory sequences include transcriptional promoters, operators, or enhancers, mRNA ribosomal binding sites, and appropriate sequences which control transcription and translation.

The choice of a suitable expression vector for expression of the polypeptide of the invention will of course depend upon the specific host cell to be used, and is within the skill of the ordinary artisan.

Expression requires that appropriate signals be provided in the vectors, such as enhancers/promoters from both viral and mammalian sources that may be used to drive expression of the nucleic acids of interest in host cells. Usually, the nucleic acid being expressed is under transcriptional control of a promoter. A "promoter" refers to a DNA sequence recognized by the synthetic machinery of the cell, or introduced synthetic machinery, required to initiate the specific transcription of a gene. Nucleotide sequences are operably linked when the regulatory sequence functionally relates to the nucleic acid sequence encoding the polypeptide of interest (i.e., a polypeptide of the invention, a derivative or fragment thereof and the like). Thus, a promoter nucleotide sequence is operably linked to a given DNA sequence if the promoter nucleotide sequence directs the transcription of the sequence.

Similarly, the phrase "under transcriptional control" means that the promoter is in the correct location and orientation in relation to the nucleic acid to control RNA polymerase initiation and expression of the gene. Any promoter that will drive the expression of the nucleic acid may be used. The particular promoter employed to control the expression of a nucleic acid sequence of interest is not believed to be important, so long as it is capable of directing the expression of the nucleic acid in the targeted cell. Thus, where a human cell is targeted, it is preferable to position the nucleic acid coding region adjacent to and under the control of a promoter that is capable of being expressed in a human cell. Generally speaking, such a promoter might include either a human or viral promoter. Common promoters include, e.g., the human cytomegalovirus (CMV) immediate early gene promoter, the SV40 early promoter, the Rous sarcoma virus long terminal repeat, [beta]-actin, rat insulin promoter, the phosphoglycerol kinase promoter and glyceraldehyde-3 -phosphate dehydrogenase promoter, all of which are promoters well known and readily available to those of skill in the art, can be used to obtain high-level expression of the coding sequence of interest. The use of other viral or mammalian cellular or bacterial phage promoters which are well-known in the art to achieve expression of a coding sequence of interest is contemplated as well, provided that the levels of expression are sufficient to produce a recoverable yield of protein of interest. By employing a promoter with well known properties, the level and pattern of expression of the protein of interest following transfection or transformation can be optimized. Inducible promoters also may be used.

Another regulatory element that is used in protein expression is an enhancer. These are genetic elements that increase transcription from a promoter located at a distant position on the same molecule of nucleic acid. Where an expression construct employs a cDNA insert, one will typically desire to include a polyadenylation signal sequence to effect proper polyadenylation of the gene transcript. Any polyadenylation signal sequence recognized by cells of the selected transgenic animal species is suitable for the practice of the invention, such as human or bovine growth hormone and SV40 polyadenylation signals.

The protein identified by the inventors seems to comprise glycosylation sites. So, the host systems preferably used are able to glycosylate the polypeptide of the invention. Those skilled in the art are able to choose such systems. Examples of host cells that can be used comprise, but are not limited to, *Lactobacillus plantarum* cells or *Lactobacillus rhamnosus.*

### Nucleic acids, vectors and host cells of the invention

A second object of the invention relates to a nucleic acid sequence encoding a polypeptide of the invention for use in the treatment or prevention of an inflammatory disease.

In a particular embodiment of the invention, said inflammatory disease is an inflammatory bowel disease.

In a preferred embodiment of the invention, said inflammatory disease is Crohn disease.

In another particular embodiment, said inflammatory disease is an inflammatory disease resulting from an activation of the NPκB pathway.

As used herein, said nucleic acid sequence may be a DNA or a RNA sequence.

In a particular embodiment of the invention, said nucleic acid sequence encodes a polypeptide comprising or consisting of the nucleic acid sequence SEQ ID NO:2-7 and SEQ ID NO:8-18.

In a preferred embodiment, said nucleic acid sequence encodes a polypeptide comprising or consisting of the nucleic acid sequence SEQ ID NO:2.

In a more preferred embodiment of the invention, said nucleic acid sequence encoding a polypeptide of the invention comprises or consists of the nucleic acid sequence defined by SEQ ID NO:19.

| | |
|---|---|
| SEQ ID NO:19 | |

As used herein, said nucleic acid sequence encompasses derivatives or fragments thereof. Preferably, said derivatives or fragments are conservative derivatives or fragments.

In a preferred embodiment, said nucleic acid sequence, derivative or fragment thereof is isolated.

A third object of the invention relates to a vector comprising a nucleic acid sequence of the invention for use in the treatment or prevention of an inflammatory disease.

In a particular embodiment of the invention, said inflammatory disease is an inflammatory bowel disease.

In a preferred embodiment of the invention, said inflammatory disease is Crohn disease.

In another particular embodiment, said inflammatory disease is an inflammatory disease resulting from an activation of the NPκB pathway.

The term "vector" (or "cloning vector" and "expression vector") means the vehicle by which a nucleic acid sequence can be introduced into a host cell, so as to transform the host and promote expression (e.g. transcription and translation) of the introduced sequence.

Typically, a nucleic acid sequence of the invention may be included in any suitable vector, such as a plasmid, cosmid, episome, artificial chromosome, phage or a viral vector.

Such vectors may comprise regulatory elements, such as a promoter, enhancer, terminator and the like, to cause or direct expression of said polypeptide upon administration to a subject. Examples of promoters and enhancers used in the expression vector for animal cell are well known in the art and include early promoter and enhancer of SV40, LTR promoter and enhancer of Moloney mouse leukemia virus, promoter and enhancer of immunoglobulin H chain and the like.

According to the invention, any expression vector for animal cell can be used, so long as a nucleic acid sequence of the invention can be inserted and expressed. Examples of suitable vectors include pAGE107, pAGE103, pHSG274, pKCR, pSG1 beta d2-4 and the like.

Other examples of plasmids include replicating plasmids comprising an origin of replication, or integrative plasmids, such as for instance pUC, pcDNA, pBR, and the like.

Other examples of viral vector include adenoviral, retroviral, herpes virus and AAV vectors. Such recombinant viruses may be produced by techniques known in the art, such as by transfecting packaging cells or by transient transfection with helper plasmids or viruses. Typical examples of virus packaging cells include PA317 cells, PsiCRIP cells, GPenv+ cells, 293 cells, etc. Detailed protocols for producing such replication-defective recombinant viruses may be found for instance in WO 95/14785, WO 96/22378, US 5,882,877, US 6,013,516, US 4,861,719, US 5,278,056 and WO94/19478.

A fourth object of the present invention relates to a host cell which has been transfected, infected or transformed by a nucleic acid sequence and/or a vector of the invention for use in the treatment or prevention of an inflammatory disease.

In a particular embodiment of the invention, said inflammatory disease is an inflammatory bowel disease.

In a preferred embodiment of the invention, said inflammatory disease is Crohn disease.

In another particular embodiment, said inflammatory disease is an inflammatory disease resulting from an activation of the NPκB pathway.

The term "transformation" means the introduction of a "foreign" nucleic acid sequence to a host cell, so that the host cell will express the introduced sequence to produce a desired substance, typically a polypeptide encoded by the introduced sequence. A host cell that receives and expresses introduced DNA or RNA bas been "transformed".

Examples of host cells that may be used for the invention are well known in the art, and some of them are described above.

The protein identified by the inventors seems to comprise glycosylation sites. So, the host cells used are preferably able to glycosylate the polypeptide of the invention. Those skilled in the art are able to choose such systems. Examples of host cells that can be used comprise, but are not limited to, *Lactobacillus plantarum* cells or *Lactobacillus rhamnosus.*

In a particular embodiment of the invention, said host cell may be a probiotic.

Said probiotic is a is a host cell, generally a bacterium or yeast cell, which has been transfected, infected or transformed by a nucleic acid sequence and/or a vector of the invention.

Examples of host cells that can be used comprise, but are not limited to, *Bacillus coagulans, Bifidobacterium animalis, Bifidobacterium longum, Lactobacillus acidophilus, Lactobacillus paracasei, Lactobacillus johnsonii, Lactobacillus plantarum, Lactobacillus reuteri, Lactobacillus reuteri Protectis, Saccharomyces boulardii, Lactobacillus rhamnosus, Lactobacillus acidophilus, Lactobacillus acidophilus, Lactobacillus bulgaricus, Streptococcus thermophiles, Lactobacillus bifidus.*

Vectors and host cells of the invention are adapted to an administration in patients, preferably humans. One skilled in the art can easily choose such vectors and host cells.

In one embodiment, the invention relates to a nucleic acid, vector or host cell of the invention for use as an anti-inflammatory drug.

According to the invention, the nucleic acid sequence, vector and host cell of the invention may be used to produce a recombinant polypeptide of the invention in a suitable expression system.

### Pharmaceutical compositions of the invention

A fifth object of the invention relates to a pharmaceutical compositions comprising a polypeptide of the invention, a nucleic acid sequence of the invention, a vector of the invention or a host cell of the invention and a pharmaceutically acceptable carrier.

In one embodiment, the invention relates to said pharmaceutical composition for the treatment of inflammatory disease.

In a more particular embodiment of the invention, said inflammatory disease is an inflammatory bowel disease.

In a preferred embodiment of the invention, said inflammatory disease is Crohn disease.

In another particular embodiment, said inflammatory disease is an inflammatory disease resulting from an activation of the NPκB pathway.

The term "pharmaceutically" or "pharmaceutically acceptable" refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

The polypeptide, nucleic acid sequence, vector or host cell of the invention may be combined with pharmaceutically acceptable excipients, and optionally sustained-release matrices, such as biodegradable polymers, to form therapeutic compositions.

In general, polypeptide, nucleic acid sequence, vector or host cell of the present invention will be administered as pharmaceutical formulations including those suitable for oral (including buccal and sub-lingual), rectal, nasal, topical, pulmonary, vaginal, or parenteral (including intramuscular, intraarterial, intrathecal, subcutaneous and intravenous) administration or in a form suitable for administration by inhalation or insufflation. The preferred manner of administration is generally oral, using a convenient daily dosage regimen which can be adjusted according to the degree of affliction.

A polypeptide, nucleic acid sequence, vector or host cell of the present invention, together with one or more conventional adjuvants, carriers, or diluents, may be placed into the form of pharmaceutical compositions and unit dosages. The pharmaceutical compositions and unit dosage forms may comprise conventional ingredients in conventional proportions, with or without additional active compounds or principles, and the unit dosage forms may contain any suitable effective amount of the active ingredient commensurate with the intended daily dosage range to be employed. The pharmaceutical compositions may be employed as solids, such as tablets or filled capsules, semisolids, powders, sustained release formulations, or liquids such as solutions, suspensions, emulsions, elixirs, or filled capsules for oral use; or in the form of suppositories for rectal or vaginal administration; or in the form of sterile injectable solutions for parenteral use. Formulations containing about one (1) milligram of active ingredient or, more broadly, about 0.01 to about one hundred (100) milligrams, per tablet, are accordingly suitable representative unit dosage forms.

The polypeptide, nucleic acid sequence, vector or host cell of the present invention may be formulated in a wide variety of oral administration dosage forms. The pharmaceutical compositions and dosage forms may comprise a polypeptide, nucleic acid sequence, vector or host cell of the present invention or pharmaceutically acceptable salts thereof as the active component. The pharmaceutically acceptable carriers may be either solid or liquid. Solid form preparations include powders, tablets, pills, capsules, cachets, suppositories, and dispersible granules. A solid carrier may be one or more substances which may also act as diluents, flavoring agents, solubilizers, lubricants, suspending agents, binders, preservatives, tablet disintegrating agents, or an encapsulating material. In powders, the carrier generally is a finely divided solid which is a mixture with the finely divided active component. In tablets, the active component generally is mixed with the carrier having the necessary binding capacity in suitable proportions and compacted in the shape and size desired. The powders and tablets preferably contain from about one (1) to about seventy (70) percent of the active compound. Suitable carriers include but are not limited to magnesium carbonate, magnesium stearate, talc, sugar, lactose, pectin, dextrin, starch, gelatin, gum tragacanth, methylcellulose, sodium carboxymethylcellulose, a low melting wax, cocoa butter, and the like. The term "preparation" is intended to include the formulation of the active compound with encapsulating material as carrier, providing a capsule in which the active component, with or without carriers, is surrounded by a carrier, which is in association with it. Similarly, cachets and lozenges are included. Tablets, powders, capsules, pills, cachets, and lozenges may be as solid forms suitable for oral administration.

Other forms suitable for oral administration include liquid form preparations including emulsions, syrups, elixirs, aqueous solutions, aqueous suspensions, or solid form preparations which are intended to be converted shortly before use to liquid form preparations. Emulsions may be prepared in solutions, for example, in aqueous propylene glycol solutions or may contain emulsifying agents, for example, such as lecithin, sorbitan monooleate, or acacia. Aqueous solutions can be prepared by dissolving the active component in water and adding suitable colorants, flavors, stabilizers, and thickening agents.

Aqueous suspensions can be prepared by dispersing the finely divided active component in water with viscous material, such as natural or synthetic gums, resins, methylcellulose, sodium carboxymethylcellulose, and other well known suspending agents. Solid form preparations include solutions, suspensions, and emulsions, and may contain, in addition to the active component, colorants, flavors, stabilizers, buffers, artificial and natural sweeteners, dispersants, thickeners, solubilizing agents, and the like.

The polypeptide, nucleic acid sequence, vector or host cell of the present invention may be formulated for parenteral administration (e.g., by injection, for example bolus injection or continuous infusion) and may be presented in unit dose form in ampoules, prefilled syringes, small volume infusion or in multi-dose containers with an added preservative. The compositions may take such forms as suspensions, solutions, or emulsions in oily or aqueous vehicles, for example solutions in aqueous polyethylene glycol. Examples of oily or nonaqueous carriers, diluents, solvents or vehicles include propylene glycol, polyethylene glycol, vegetable oils (e.g., olive oil), and injectable organic esters (e.g., ethyl oleate), and may contain formulatory agents such as preserving, wetting, emulsifying or suspending, stabilizing and/or dispersing agents.

Alternatively, the active ingredient may be in powder form, obtained by aseptic isolation of sterile solid or by lyophilization from solution for constitution before use with a suitable vehicle, e.g., sterile, pyrogen-free water.

The polypeptide, nucleic acid sequence, vector or host cell of the present invention may be formulated for topical administration to the epidermis as ointments, creams or lotions, or as a transdermal patch. Ointments and creams may, for example, be formulated with an aqueous or oily base with the addition of suitable thickening and/or gelling agents. Lotions may be formulated with an aqueous or oily base and will in general also containing one or more emulsifying agents, stabilizing agents, dispersing agents, suspending agents, thickening agents, or coloring agents. Formulations suitable for topical administration in the mouth include lozenges comprising active agents in a flavored base, usually sucrose and acacia or gum tragacanth; pastilles comprising the active ingredient in an inert base such as gelatin and glycerin or sucrose and acacia; and mouthwashes comprising the active ingredient in a suitable liquid carrier.

The polypeptide, nucleic acid sequence, vector or host cell of the present invention may be formulated for administration as suppositories. A low melting wax, such as a mixture of fatty acid glycerides or cocoa butter is first melted and the active component is dispersed homogeneously, for example, by stirring. The molten homogeneous mixture is then poured into convenient sized molds, allowed to cool, and to solidify.

The polypeptide, nucleic acid sequence, vector or host cell of the present invention may be formulated for vaginal administration.

Pessaries, tampons, creams, gels, pastes, foams or sprays containing in addition to the active ingredient such carriers as are known in the art to be appropriate.

The polypeptide, nucleic acid sequence, vector or host cell of the present invention may be formulated for nasal administration. The solutions or suspensions are applied directly to the nasal cavity by conventional means, for example, with a dropper, pipette or spray. The formulations may be provided in a single or multidose form. In the latter case of a dropper or pipette, this may be achieved by the patient administering an appropriate, predetermined volume of the solution or suspension. In the case of a spray, this may be achieved for example by means of a metering atomizing spray pump.

The polypeptide, nucleic acid sequence, vector or host cell of the present invention may be formulated for aerosol administration, particularly to the respiratory tract and including intranasal administration. The compound will generally have a small particle size for example of the order of five (5) microns or less.

Such a particle size may be obtained by means known in the art, for example by micronization. The active ingredient is provided in a pressurized pack with a suitable propellant such as a chlorofluorocarbon (CFC), for example, dichlorodifluoromethane, trichlorofluoromethane, or dichlorotetrafluoroethane, nitrogen, nitrous oxide, carbon dioxide or other suitable gas. The aerosol may conveniently also contain a surfactant such as lecithin. The dose of drug may be controlled by a metered valve. Alternatively the active ingredients may be provided in a form of a dry powder, for example a powder mix of the compound in a suitable powder base such as lactose, starch, starch derivatives such as hydroxypropylmethyl cellulose and polyvinylpyrrolidine (PVP). The powder carrier will form a gel in the nasal cavity. The powder composition may be presented in unit dose form for example in capsules or cartridges of e.g., gelatin or blister packs from which the powder may be administered by means of an inhaler.

When desired, formulations can be prepared with enteric coatings adapted for sustained or controlled release administration of the active ingredient. For example, the compounds of the present invention can be formulated in transdermal or subcutaneous drug delivery devices. These delivery systems are advantageous when sustained release of the compound is necessary and when patient compliance with a treatment regimen is crucial. Compounds in transdermal delivery systems are frequently attached to a skin-adhesive solid support.

Other pharmaceutically acceptable forms include, e.g. tablets or other solids for oral administration; time release capsules; and any other form currently used. Non degradable capsules, or gastro-resistant capsules may also be used. Such pharmaceutical forms are well known in the art.

In certain embodiments, the use of liposomes and/or nanoparticles is contemplated for the administration of polypeptide, nucleic acid sequence, vector or host cell of the present invention. Liposomes are particularly suitable for an oral administration of a hydrophobic compound. The formation and use of liposomes and/or nanoparticles are known to those of skill in the art.

In the particular embodiment of the treatment of an inflammatory bowel disease, more particularly Crohn disease, an oral or a rectal administration are preferred. For oral administration, gastro-resistant, non degradable and time release capsules are preferred.

In the particular embodiment of a composition of the invention comprising a host cell of the invention which is a probiotic, the composition may be used by oral administration.

In general, the polypeptide, nucleic acid sequence, vector or host cell of the present invention will be administered in a therapeutically effective amount by any of the accepted modes of administration for agents that serve similar utilities. Suitable dosage ranges are typically about 1-500 mg daily, preferably about 1-100 mg daily, and most preferably about 1-30 mg daily, depending upon numerous factors such as the severity of the disease to be treated, the age and relative health of the subject, the potency of the compound used, the route and form of administration, the indication towards which the administration is directed, and the preferences and experience of the medical practitioner involved. One of ordinary skill in the art of treating such diseases will be able, without undue experimentation and in reliance upon personal knowledge and the disclosure of this Application, to ascertain a therapeutically effective amount of the compounds of the present invention for a given disease.

The pharmaceutical preparations are preferably in unit dosage forms. In such form, the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.

### EXAMPLES

The following examples describe some of the preferred modes of making and practicing the present invention. However, it should be understood that the examples are for illustrative purposes only and are not meant to limit the scope of the invention.

### Example 1. Identification of the molecule(s) responsible for the anti-inflammatory effect of Faecalibacterium prausnitzii.

### 1. Identification of six peptides only present in the supernatant of F. prausnitzii.

Further to the knowledge of SOKOL et al., PNAS, 2008, the present inventors first tried to identify the molecules responsible for the anti-inflammatory effect of *Faecalibacterium prausnitzii.* Nevertheless, this determination was clearly unpredictable because of the difficulty to cultivate *F. prausnitzii,* which microorganism is strictly anaerobic. Moreover, the characterization of this microorganism being very recent, its characteristics, specificities, taxonomy, etc. are essentially unknown further complicating its use as an experimental model.

### 1.1. culture ofF. prausnitzii

The culture of the F. prausnitzii was difficult, in part due to the sensitivity of the bacterium and to the necessity of anaerobia conditions.

### 1.2. Fractionation of the F. prausnitzii culture medium

A solid/liquid extraction of 3 ml of medium on Waters Oasis HLB® SPE cartridges was carried out to fractionated culture supernatant. Six fractions were obtained by eluting with 20%, 30%, 45%, 60%, 90% and 100% of acetonitrile. These fractions were dried using a speed-vac, then, taken with DMEM. Tests on Caco-2 cells stimulated by IL-1, showed an anti-inflammatory effect for a fraction of supernatant eluted with 90% acetonitrile.

### 1.3. Comparative mass spectrometry analysis of F. prausnitzii culture medium.

The "active" fractions eluted with 90% of acetonitrile were then analysed to detect molecules specific of the culture supernatant of *F. prausnitzii.* The MALDI-TOF mass spectra of LyBHI and culture supernatant were obtained from Voyager® DE Pro (AB-Sciex) in the linear and reflector positive mode, using a nitrogen UV laser (337 nm, laser 3 Hz, pulse 3 ns) and a matrix of 100 mM α-cyano-4-hydroxycinnamic acid (CHCA) in 70/30 % (v/v) acetonitrile/water TFA 0.1 % solution. Spectra were obtained with an extraction delay of 125 ns (for the reflector mode) or 300 ns (for the linear mode), an accelerating voltage of 20 kV and an averaging of 300 laser shots per sample. The mass range studied is from 500 to 4000 Da for the reflector mode and from 3500 to 30 000 Da for the linear mode.

Six ions (m/z 1733,93 ; 1832,92; 1946,97; 2047,95, 2146,94 et 4601,06) were identified in the *F. prausnitzii* supernatant fraction eluted with 90% of acetonitrile. No difference between the culture medium LyBHI and the *F. prausnitzii* supernatant was observed in the other fractions of medium.

### 1.4. Identification of molecules by a FT-ICR mass spectrometer.

The ions of interest were fragmented by a FT-ICR mass spectrometer, equipped with a 7T superconducting magnet (Apex Qe®, Bruker Daltonics, Bremen, Germany). Mass spectral data were acquired in the broadband mode over an *m*/*z* range of 150 to 1500 with 256 k data points, leading to the registration of a transient signal of 0.17 s. Data processing and data acquisition were performed using respectively Apex Control® version 2.1 software and Data Analysis® version 3.4 (Bruker Daltonics). Mass values displayed correspond to the monoisotopic masses. The mass spectra obtained for these six ions correspond to spectra of fragmentation peptides. Thus, a *de novo* sequencing of the six peptides was necessary to obtain a sequence of amino acids.

### 1.5. De novo sequencing of the six peptides.

The freeze-dried fractions were resuspended in a mixture methanol/water containing, 0.5% formic acid and directly infused into the hybrid mass spectrometer Qh-FT/ICR. Mass spectra were recorded in positive mode ionization. The kinetic energy was 4 eV for the registration of a mass spectrum and about 15 eV for a spectrum of product ions. The accumulation time of ions in the collision cell, acting as a linear trap, were 0.5 s. The transfer time of ions between the collision cell and the ICR cell was set at 0.001 sec. The ions were trapped in the ICR cell by a trapping potential of 2V, and this potential is reduced to 1 V for the step excitation/detection.

Here are the sequences of amino acids obtained by *de novo* sequencing:

| | |
|---|---|
| Peptide 1 | GNTF[I/L]QST[I/L]NRT[I/L]GV[I/L] |
| Peptide 2 | VGNTF[I/L]QST[I/L]NRT[I/L]GV[I/L] |
| Peptide 3 | [I/L]VGNTF[I/L]QST[I/L]NRT[I/L]GV[I/L] |
| Peptide 4 | T[I/L]VGNTF[I/L]QST[I/L]NRT[I/L]GV[I/L] |
| Peptide 5 | VT[I/L]VGNTF[I/L]QST[I/L]NRT[I/L]GV[I/L] |
| Peptide 6 | FSGNTTWKEVGNIGKNLFGTNVKGNPIEKNNFGDYAMNALGIA |

*Peptide 1 corresponds to the peptide defined by the sequence SEQ ID NO:11 described above, peptide 2 corresponds to the peptide defined by the sequence SEQ ID NO:12 described above, peptide 3 corresponds to the peptide defined by the sequence SEQ ID NO:13 described above, peptide 4 corresponds to the peptide defined by the sequence SEQ ID NO:14 described above, peptide 5 corresponds to the peptide defined by the sequence SEQ ID NO:15 described above, peptide 6 corresponds to the peptide defined by the sequence SEQ ID NO:10 described above.*

*The identification of residues leucine and isoleucine were initially undefined because they are isomeric amino acids and the genome of F. prausnitzii was not sequenced.*

### I.5. In silico analysis of the six peptides

As soon as the genome of *F.prausnitzii* was sequenced, the in *silico* analysis (NCBInr) allowed to confirm that these six peptides are all derived from one *F. prausnitzii* A2-165 protein ZP 05614546.1 (Accession number changed to WP_005932151; SEQ ID NO:2) conserved hypothetical protein (E-value: 10⁻⁹) and to remove the ambiguity between leucine and isoleucine.

This analysis also allowed to detect a posteriori three other peptides from this protein ZP 05614546.1 (Accession number changed to WP_005932151; SEQ ID NO:2) in this active fraction, with a very low intensity on the MS-spectra.

| | |
|---|---|
| Peptide 7 | NTFLQSTINRTIGVL |
| Peptide 8 | AAVYNLGVAPTKNTVKETEVKFTV |
| Peptide 9 | NYSVIAENEMTYVNGGANFIDAIGAVTAPIWTLDNVKTFNTNIVT LV |

*Peptide 7 corresponds to the peptide defined by the sequence SEQ ID NO:16 described above, peptide 8 corresponds to the peptide defined by the sequence SEQ ID NO:17 described above, peptide 9 corresponds to the peptide defined by the sequence SEQ ID NO:18 described above.*

In this first part, the inventors identified nine peptides which were detected only in the supernatant of *F. prausnitzii.* These peptides were present in eluted active fraction from the supernatant of *F. prausnitzii,* and absent from control fractions, without inflammatory effect.

The inventors thus identified anti-inflammatory peptides, all issued from a protein (ZP 05614546.1, Accession number changed to WP_005932151; SEQ ID NO:2) of 14 491,350 Da synthesized by *F. prausnitzii.* The function of this protein is unknown.

### 2. Evaluation of the anti-inflammatory effects of the protein ZP05614546.1 (Accession number changed to WP_005932151; SEQ ID NO:2) and its derived peptides.

In order to confirm that anti-inflammatory effects of *F. prausnitzii* supernatant relies at least in part on ZP05614546.1 and/or its derived peptides, the inventors tested synthetic protein derived peptides and the ZP05614546.1 protein itself using two approaches.

### 2.1. Characterization of the protein

The inventors studied the different properties of the protein.

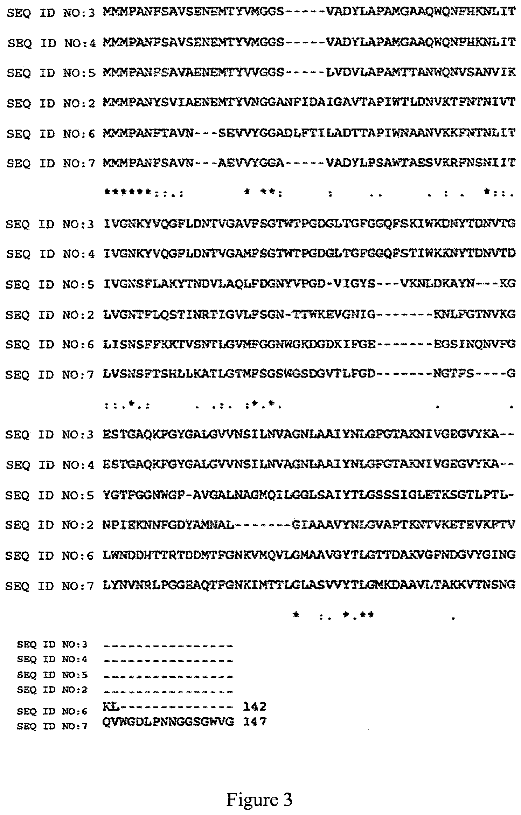

First, they identified by alignment on biological databases five orthologs sequences having a significant identity with the sequence of ZP05614546.1. Said sequences seem to belong to other strains of *F. prausnitzii.* These sequences may certainly correspond to orthologs of the protein ZP05614546.1 (Figure 3). A consensus sequence was obtained from this alignment corresponding to SEQ ID NO:1.

Interestingly, they noted that the N-terminal part of the protein (about the 80 first amino acids; SEQ ID NO: 8) is highly conserved. A central part of the protein also shows a significant conservation for about 20 amino acids around the 150^{th} amino acids.

Studying the hypothetical structure of the protein, they show that it contains two hydrophobic domains around the position AA20-50 and AA90-120. This second hydrophobic domains corresponds the 20aa domain significantly conserved around the 150^{th} amino acids. Another structural analysis of the sequence shows two hypothetical α-helical regions matching with these two hydrophobic domains.

Based on these analyses, the core structure of the polypeptide of the invention corresponds to an amino acid sequence having, in the N-term to C-term orientation:
- the consensus sequence SEQ ID NO:8; and
- the consensus sequence SEQ ID NO:9,
wherein the sequence SEQ ID NO:8 and SEQ ID NO:9 are preferably spaced from 80 to 10 amino acids, preferably from 60 to 15 amino acids, and more preferably from 40 to 20 amino acids.

Interestingly, the study of the sequence and structure of the protein seems to show that it does not contain any signal peptide. Some of the fragments identified by the inventors (as described above) correspond to fragments of the conserved regions within the five "orthologs" sequences.

### 2.2. Direct incubation of intestinal epithelial cell models with protein ZP05614546.1 and the derived peptides of interest

The inventors thus incubated intestinal epithelial HT29 cells with the protein and/or with the derived peptides described above.

Using said cells, they did not obtain any result that could show any anti-inflammatory effect using this strategy.

The inventors then incubated mucus secreting intestinal epithelial HT29-MTX cells with two peptide fragments obtained from the protein ZP05614546.1.

| | |
|---|---|
| SEQ ID NO:20 | KGNTFLQSTINRTIGVL |
| SEQ ID NO:21 | |

These both peptides defined by the amino acid sequences SEQ ID NO:20 and SEQ ID NO:21 respectively correspond to the regions between positions 53 and 68 and 90 and 135 of the protein ZP05614546.1

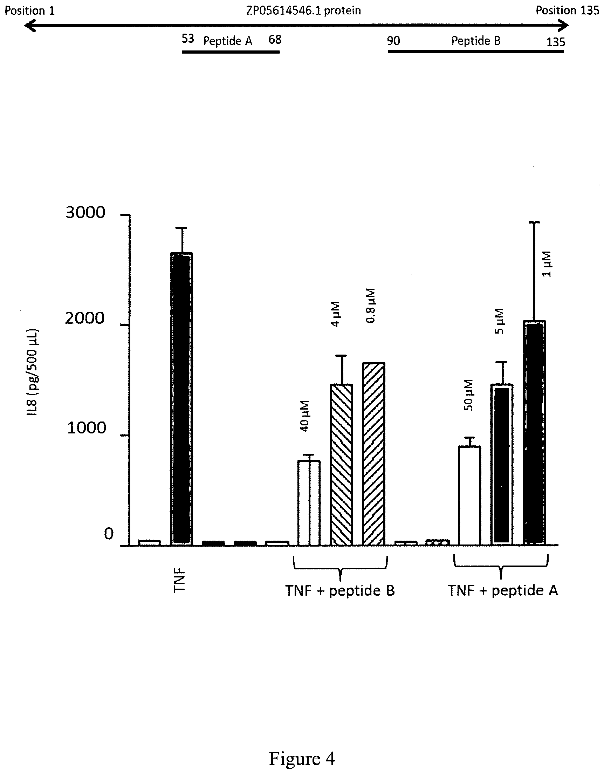

The inventors measured the IL-8 production (as a marker of inflammation) after stimulation of HT29-MTX cells incubated or not with said peptides and stimulated by TNFα. The results show that these both peptides have an anti-inflammatory effect in a dose dependent manner (figure 4).

This biological activity observed with the peptides in the HT29-MTX cell model may result from an interaction between the polypeptides of the invention and the mucus.

### 2.3. Transfection of protein ZP05614546.1 into epithelial cell models

In order to further analyze the activity of the polypeptides of the invention, the inventors decided to transfect the protein-coding cDNA directly into mammalian epithelial cell model.

The sequence of the protein ZP05614546.1 has been cloned in 3xFlag (C-term) pCMV vector.

Using an NPκB reporter system in human epithelial cells HEK 293T, they showed that the protein ZP05614546.1 inhibits the NPκB signaling pathway in a dose-dependent manner.

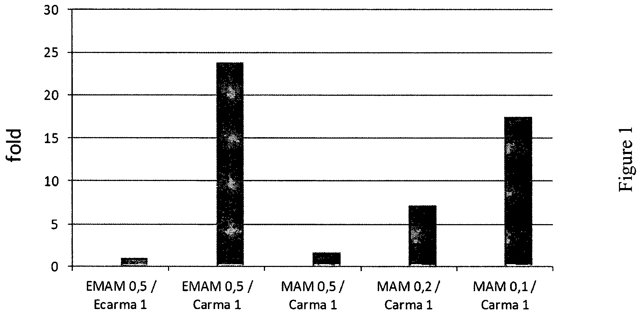

Indeed, the protein ZP05614546.1 inhibits Carma-1-dependent NPκB activation (Figure 1).

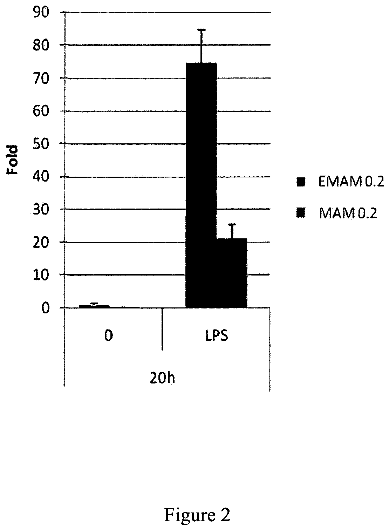

In HEK293T cells stably expressing TLR4, MD2 and CD14, the inventors further showed that the protein ZP05614546.1 inhibits the LPS-dependent NPκB activation (Figure 2).

Expression of ZP05614546.1 was confirmed in HEK293T cells by Western blot with anti-Flag antibody. In another cell model (HeLa), the expression was validated by immunofluorescence and ZP05614546.1 was localized around the cell nucleus.

In conclusion, the inventors showed that, even if the first results seem to show the contrary, the protein ZP05614546.1 and its derived peptides described above have an anti-inflammatory effect.

### Example 2. Use of anti-inflammatory effects of the protein of the invention

### Constructions

Secreted form of the protein ZP05614546.1 was expressed in a NICE (Nisine Induced Controlled Expression) host system (*L. lactis* strain NZ9000), using a expression cassette pSEC wherein the gene encoding ZP05614546.1 is cloned downstream to the pNis promoter and a Usp45signal peptide. The plasmid harbors a resistance gene to chloramphenicol.

The gene encoding ZP05614546.1 is produced by synthesis in order to optimize the codons for *L. lactis* for a better expression of the protein in this host cell. The gene is digested by restriction enzymes compatible with those digesting the pSEC vecteur (NsiI, SpeI). The ligation between the vector and the insert generates a plasmid which is introduced into the NZ9000 strain by electroporation.

The clones that grow on a medium comprising M17 medium + glucose 0.5% + Chloramphenicol 10µg/mL are analyzed for the presence of the insert coding for the protein ZP05614546.1. The purified plasmid comprising the insert is sequenced in order to confirm the integrity of the nucleic acid sequence encoding the protein of interest in said plasmid. After its sequence confirmed, the plasmid is transferred in strains NZ9000 (Htra-), NZ9000 (Clp-) and NZ9000 (Htra-/Clp-).

The strains that contain the plasmid encoding the protein ZP05614546.1 is reintroduced on a medium comprising M17 + glucose 0.5% + Chloramphenicol 10µg/mL and incubated during the night at 30°C. The morning after that, the strain is diluted at 1/100. Expression of the protein is induced with nisine at 10ng/mL during one hour at 30°C. A protein extraction is further realized by separating the centrifugate and the supernatant and by treating them differently. The proteins present in the supernatant are precipitated with TCA and centrifuged during 30 min, and then they are introduced in a Laemmli buffer. The centrifugate is mixed in PBS comprising antiprotease and is sonicated at a 6*10 sec cycle. The lysate is centrifuged in order to eliminate the bacterium fragments and the supernatant which contains a lot of proteins is kept for further studies.

The construction may be realized in another plasmid (an optimized plasmid similar to the previous one) that permits a better secretion of the protein of interest: the presence of a nucleic aid sequence encoding a polypeptide of 9 amino acids (LEISSTCD, SEQ ID NO:22) placed between the nucleic acid sequence encoding the signal peptide and the nucleic acid sequence encoding the protein of interest adds two negative charges to the protein, allowing a better transport through the bacterium membrane.

Cloning is done by introduction of the nucleic acid sequence encoding the protein of interest in such a plasmid as described above. Said plasmid is introduced in the NZ9000 WT strain and in the NZ9000 Htra- strain (deficient in its extern protease).

Another construction is envisaged, that permits a cytoplasmic form of the protein of interest. It consists in the fusion of the nucleic acid sequence encoding the protein of interest with the promoter pNis without the Usp45 signal peptide. The cytoplasmic form of said protein allows the protection of the protein in hard extern conditions.

Cloning and expression of the protein are made as describe above. The supernatant of bacteria expressing the proteins is not analyzed as the protein is not anymore secreted.

### In vitro studies of the anti-inflammatory effects of the protein and its derived fragments.

HT29 epithelial cells are in a DMEM medium comprising 10% serum SVF and 1% glutamine at a concentration of 0.1 10⁶ cells/mL. The cells are plated in 24-wells plates (500µL of medium by well). The plates are placed at 37°C in a 10% CO₂ sterilizing room during 72h.

Culture medium is changed 3 times and cells incubated during 24h each time. Then, cells are stressed in a DMEM medium comprising 5% serum SVF and 1% glutamine is added (500µL by well). The plates are placed at 37°C in a 10% CO₂ sterilizing room during 24h.

After that, HT29 cells are co-incubated with bacteria expressing the protein ZP05614546.1 (at a ratio 1/40) and supernatants are recovered.

Media comprises DMEM, 5% SFV serum, 1% glutamine, with or without TNFα at a concentration of 5ng/µL, with or without antibiotics PS 0.1%.

Bacteria cultures are centrifuged a 3000g during 5 min, centrifugates are recovered in 1 mL DMEM and centrifuged again at 3000g during 5 min. Centrifugates are recovered in 1 mL DMEM + 5% SVF serum + 1% glutamine.

DMEM is eliminated, 450 µL/well of medium comprising DMEM + 5% SVF serum + 1% glutamine + 0.1% PS or DMEM + 5% SVF serum +1% glutamine+0.1% PS + TNFα is added. 50µL of bacterium solution. The control does not comprise bacteria solution.

Plates are placed in CO2 sterilizing room at 37°C during 6h.

Supernatant of cultures are recovered and stocked.

### In vivo studies of the anti-inflammatory effects of the protein and its derived fragments.

C57B16 mice (6-8 weeks old) are kept at room temperature, under 12h light/dark cycles and having free access to food and water, except the day before the induction of colitis, where they are fasted for 12h.

Colonic inflammation is induced by treatments with Dextran Sodium Sulfate (DSS). In details, DSS is dissolved in drinking water (3 or 5% wt/vol) and the animals are free to drink this solution for 7-days. Water consumption is measured in the DSS-treated groups and compared to groups of naive mice drinking water: no difference is observed for the volume of liquid consumed, between water and DSS-drinking mice. Mice are treated daily orally, with 200 µL of 1 to 5.10⁹ colony forming units (cfu) of strains to be tested or bacterial milieu alone -i.e. *L. lactis* expressing the protein ZP 05614546.1 (Accession number changed to WP_005932151) or not. The first treatment starts at the same time DSS is added to drinking water (but it can also be performed in advance, as a preventive treatment) and the last treatment is on the day of the sacrifice (day 7). Body weight and survival rate are measured daily after the induction of colitis (or right after the first gavage, in case of preventive treatment). DAI (disease activity index is also measured):

The Disease activity index (Cooper HS Lab Invest 1993;69:238-49) is carried out each day (as showed in the table below).

| | Description | Score |
|---|---|---|
| Diarrhea | Normal | 0 |
| | Soft | 2 |
| | Liquid | 4 |
| Blood in faeces | No | 0 |
| | Low | 2 |
| | important | 4 |
| Loose of weight | <1% | 0 |
| | 1-5% | 1 |
| | 5-10% | 2 |
| | 10-15% | 3 |
| | >15% | 4 |
| Activity Score | | Sum of the 3 scores |

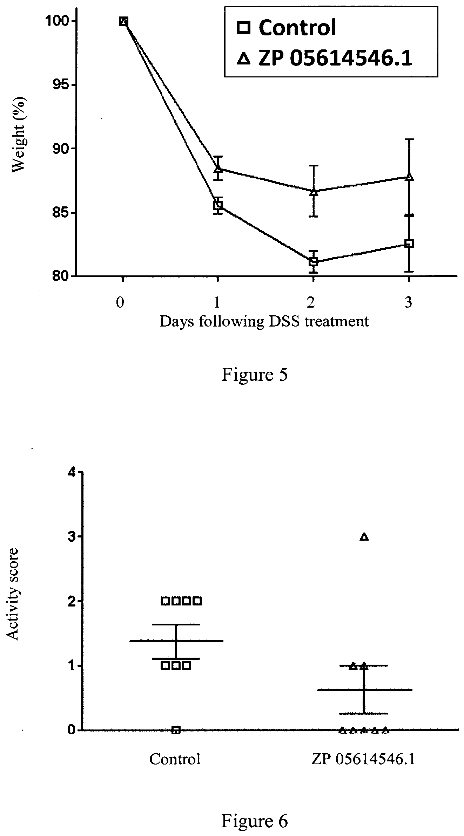

The figure 5 shows the body weight evolution in the DSS inflammation model for control mice (□) and ZP 05614546.1 daily treated mice (△).

The figure 6 shows the activity score observed at day 3 following colonic inflammation induction by DSS for control mice (□) and ZP 05614546.1 daily treated mice (△).

On day 7 after adding DSS to their drinking water, mice are sacrificed and colons are harvested for measure of several parameters of inflammation: bowel thickness and length, myeloperoxydase (MPO) activity, cytokine expression (IL-17A, IFN-γ, IL10, IL12p70 or other cytokines) can be measured. Colon washes are also performed with 1ml of PBS+ anti-protease cocktail. 400-500µl of blood are collected for cytokine analysis.

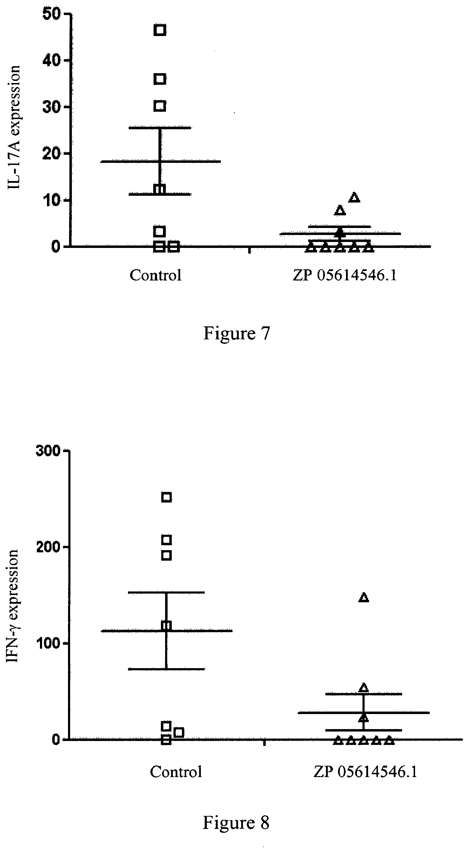

Thes figure 7 and figure 8 show the IL-17A and IFN-γ expression respectively for control mice (□) and ZP 05614546.1 daily treated mice (△).

Finally, the *in vivo* results confirm the previous *in vitro* results establishing that the ZP 05614546.1 protein significantly reduce colonic inflammation.

## Claims

1. A polypeptide comprising or consisting of the amino acid sequence SEQ ID NO: 1, apolypeptide having at least 90% sequence identity with SEQ ID NO:1 and retaining the anti-inflammatory effect of SEQ ID NO:1 or a fragment of said polypeptides having a length of at least 10 amino acids and retaining the anti-inflammatory effect of SEQ ID NO: 1, for use in the treatment or prevention of an inflammatory disease.

2. The polypeptide for use according to claim 1, wherein said inflammatory disease is an inflammatory bowel disease, chosen among Crohn disease, ulcerative colitis, ileitis and enteritis.

3. The polypeptide for use according to any one of claims 1-2, wherein said inflammatory disease is Crohn disease.

4. The polypeptide for use according to any one of claims 1-3, wherein said polypeptide comprises or consists of the amino acid sequence SEQ ID NO:2-7.

5. The polypeptide for use according to any one of claims 1-3, wherein said conservative fragment of the invention comprises or consists of a polypeptide chosen among SEQ ID NO:8-18.

6. The polypeptide for use according to any one of claims 1-3, wherein said polypeptide corresponds to an amino acid sequence having in the N-term to C-term orientation:
- the sequence SEQ ID NO:8; and
- the sequence SEQ ID NO:9,
wherein the sequence SEQ ID NO:8 and SEQ ID NO:9 are preferably spaced from 80 to 10 amino acids, preferably from 60 to 15 amino acids, and more preferably from 40 to 20 amino acids.

7. A nucleic acid sequence encoding a polypeptide according to any one of claims 1-6 for use in the treatment or prevention of an inflammatory disease.

8. The nucleic acid sequence for use according to claim 7, wherein said nucleic acid sequence encoding a polypeptide of the invention comprises or consists of the nucleic acid sequence defined by SEQ ID NO:19.

9. A vector comprising a nucleic acid sequence for use according to any one of claims 7-8, for use in the treatment or prevention of an inflammatory disease.

10. A host cell which has been transfected, infected or transformed by a nucleic acid sequence according to any one of claims 7-8 and/or a vector according to claim 9, for use in the treatment or prevention of an inflammatory disease.

11. A pharmaceutical composition comprising a polypeptide according to any one of claims 1-5, a nucleic acid sequence according to any one of claims 7-8, a vector according to claim 9 or a host cell according to claim 10 and a pharmaceutically acceptable carrier.

12. A pharmaceutical composition comprising a polypeptide according to any one of claims 1-5, a nucleic acid sequence according to any one of claims 7-8, a vector according to claim 9 or a host cell according to claim 10 and a pharmaceutically acceptable carrier for use in the treatment of an inflammatory disease.

13. The pharmaceutical composition for use according to claim 12, wherein said inflammatory disease is an inflammatory bowel disease.

14. The pharmaceutical composition for use according to any one of claims 12-13, wherein said inflammatory disease is Crohn disease.

15. The pharmaceutical composition for use according to claim 12, wherein said inflammatory disease is an inflammatory disease resulting from an activation of the NFκB pathway.

## Patentansprüche

1. Ein Polypeptid, die Aminosäuresequenz SEQ ID NO:1 aufwelsend oder daraus bestehend, ein Polypeptid mit einer Sequenzidentität von mindestens 90% mit SEQ ID NO:1 und die entzündungshemmende Wirkung von SEQ ID NO:1 bewahrend oder ein Fragment der genannten Polypeptide mit einer Länge von mindestens 10 Aminosäuren und die entzündungshemmende Wirkung von SEQ ID NO:1 bewahrend, zum Gebrauch in der Behandlung oder Vorbeugung einer Entzündungskrankheit.

2. Polypeptid zum Gebrauch nach Patentanspruch 1, wobei die genannte Entzündungskrankheit eine entzündliche Darmkrankhelt unter Crohn-Krankhett, Colitis ulcerosa, Ileltis und Enteritis Ist.

3. Polypeptid zum Gebrauch nach Irgendeinem der Patentansprüche 1 bis 2, wobei die genannte Entzündungskrankhelt Crohn-Krankheit ist.

4. Polypeptid zum Gebrauch nach Irgendeinem der Patentansprüche 1 bis 3, wobei das genannte Polypeptid die Aminosäuresequenz SEQ ID NO:2-7 umfasst oder daraus besteht.

5. Polypeptid zum Gebrauch nach Irgendeinem der Patentansprüche 1 bis 3, wobei das genannte erfindungsgemäße bewahrende Fragment ein Polypeptid unter SEQ ID NO:8-18 umfasst oder daraus besteht.

6. Polypeptid zum Gebrauch nach irgendeinem der Patentansprüche 1 bis 3, wobei das genannte Polypeptid
einer Aminosäuresequenz entspricht, die In der Richtung vom N-Ende zum C-Ende
- die Sequenz SEQ ID NO:8 und
- die Sequenz SEQ ID NO:9 aufweist,
wobei sich die Sequenzen SEQ ID NO:8 und SEQ ID NO:9 vorzugsweise in einem Abstand von 80 bis 10 Aminosäuren, vorzugsweise in einem Abstand von 60 bis 15 Aminosäuren und besonders bevorzugt von 40 bis 20 Aminosäuren voneinander befinden.

7. Nukleinsäuresequenz, die ein Polypeptid nach Irgendeinem der Patentansprüche 1-6 zum Gebrauch In der Behandlung oder Vorbeugung einer Entzündungskrankhell codiert.

8. Nukleinsäuresequenz zum Gebrauch nach Patentanspruch 7, wobei die genannte ein erfindungsgemäßes Polypeptid codierende Nukleinsäuresequenz die Nukleinsäuresequenz umfasst oder daraus besteht, die durch SEQ ID NO:19 definiert wird.

9. Vektor, eine Nukleinsäuresequenz, zum Gebrauch nach irgendeinem der Patentansprüche 7-8 aufweisend, zum Gebrauch In der Behandlung oder Vorbeugung einer Entzündungskrankhelt.

10. Wirtszelle, die mit einer Nukleinsäuresequenz nach irgendeinem der Patentansprüche 7-8 und/oder einem Vektor nach Patentanspruch 9 transfiziert, infiziert oder transformiert wurde, zum Gebrauch in der Behandlung oder Vorbeugung einer Entzündungskrankheit.

11. Pharmazeutische Zubereitung, ein Polypeptid nach Irgendeinem der Patentansprüche 1 bis 5 enthaltend, eine Nukleinsäuresequenz nach irgendeinem der Patentansprüche 7-8, einen Vektor nach Patentanspruch 9 oder eine Wirtszelle nach Patentanspruch 10, sowie einen pharmazeutisch verträglichen Träger.

12. Pharmazeutische Zubereitung, ein Polypeptid nach irgendeinem der Patentansprüche 1 bis 5 enthaltend, eine Nukleinsäuresequenz nach Irgendeinem der Patentansprüche 7-8, einen Vektor nach Patentanspruch 9 oder eine Wirtszelle nach Patentanspruch 10, sowie einen pharmazeutisch verträglichen Träger, zum Gebrauch In der Behandlung einer Entzündungskrankheit.

13. Pharmazeutische Zubereitung zum Gebrauch nach Patentanspruch 12, wobei die genannte Entzündungskrankheit eine entzündliche Darmkrankheit ist.

14. Pharmazeutische Zubereitung zum Gebrauch nach irgendeinem der Patentansprüche 12 bis 13, wobei die genannte Entzündungskrankheit Crohn-Krankhelt Ist.

15. Pharmazeutische Zubereitung zum Gebrauch nach Patentanspruch 12, wobei die genannte Entzündungskrankheit eine Entzündungskrankheit Ist, die durch Aktivlerung des NPκB-Signalweges entsteht.

## Revendications

1. Un polypeptide comprenant ou consistant en la séquence d'acide aminé SEQ ID NO:1, un polypeptide ayant au moins 90% d'identité de séquence avec SEQ ID NO:1 et présentant l'effet anti-inflammatoire de SEQ ID NO:1 ou un fragment desdits polypeptides ayant une longueur d'au moins 10 acides aminés et présentant l'effet anti-inflammatoire de SEQ ID NO: 1, pour une utilisation dans le traitement ou la prévention d'une pathologie inflammatoire.

2. Le polypeptide pour une utilisation selon la revendication 1, dans laquelle ladite pathologie inflammatoire est une maladie inflammatoire de l'intestin choisie parmi la maladie de Crohn, la rectocolite hémorragique, l'iléite et l'entérite.

3. Le polypeptide pour une utilisation selon l'une quelconque des revendications 1 ou 2, dans laquelle ladite pathologie inflammatoire est la maladie de Crohn.

4. Le polypeptide pour une utilisation selon l'une quelconque des revendications 1 à 3, dans laquelle ledit polypeptide comprend ou consiste en la séquence d'acide aminé SEQ ID NO:2-7.

5. Le polypeptide pour une utilisation selon l'une quelconque des revendications 1 à 3, dans laquelle ledit fragment conservatif comprend ou consiste en un polypeptide choisi parmi SEQ ID NO:8-18.

6. Le polypeptide pour une utilisation selon l'une quelconque des revendications 1 à 3, dans laquelle ledit polypeptide correspond à une séquence d'acide aminé ayant, dans l'orientation N-terminal vers C-terminal :
- la séquence SEQ ID NO:8; et
- la séquence SEQ ID NO:9,
dans laquelle les séquences SEQ ID NO:8 et SEQ ID NO:9 sont préférentiellement espacées de 80 à 10 acides aminés, de préférence de 60 à 15 acides aminés, et plus préférentiellement de 40 à 20 acides aminés.

7. Une séquence d'acide nucléique codant pour un polypeptide selon l'une quelconque des revendications 1 à 6 pour une utilisation dans le traitement ou la prévention d'une pathologie inflammatoire.

8. La séquence d'acide nucléique pour une utilisation selon la revendication 7, dans laquelle ladite séquence d'acide nucléique codant pour un polypeptide selon l'invention comprend ou consiste en la séquence d'acide nucléique définie par SEQ ID NO: 19.

9. Un vecteur comprenant la séquence d'acide nucléique pour une utilisation selon l'une quelconque des revendications 7-8, pour une utilisation dans le traitement ou la prévention d'une pathologie inflammatoire.

10. Une cellule hôte qui a été transfectée, infectée ou transformée par une séquence d'acide nucléique selon l'une quelconque des revendications 7-8 et/ou un vecteur selon la revendication 9, pour une utilisation dans le traitement ou la prévention d'une pathologie inflammatoire.

11. Une composition pharmaceutique comprenant un polypeptide selon l'une quelconque des revendications 1-5, une séquence d'acide nucléique selon l'une quelconque des revendications 7-8, un vecteur selon la revendication 9 ou une cellule hôte selon la revendication 10 et un support pharmaceutiquement acceptable.

12. Une composition pharmaceutique comprenant un polypeptide selon l'une quelconque des revendications 1-5, une séquence d'acide nucléique selon l'une quelconque des revendications 7-8, un vecteur selon la revendication 9 ou une cellule hôte selon la revendication 10 et un support pharmaceutiquement acceptable pour une utilisation dans le traitement d'une pathologie inflammatoire.

13. La composition pharmaceutique pour une utilisation selon la revendication 12, dans laquelle ladite pathologie inflammatoire est une maladie inflammatoire de l'intestin.

14. La composition pharmaceutique pour une utilisation selon l'une quelconque des revendications 12-13, dans laquelle ladite pathologie inflammatoire est la maladie de Crohn.

15. La composition pharmaceutique pour une utilisation selon la revendication 12, dans laquelle ladite pathologie inflammatoire résulte d'une activation de la voie NFκB.
